# EUROPEAN PATENT APPLICATION

(11) **EP 1 632 565 A1**
(43) Date of publication of application: **08.03.2006**
(21) Application number: 04019154.6
(22) Date of filing: 12.08.2004
(51) Int. Cl.: C12N 9/02, C12N 15/82, A01H 5/00

(54) **Modulation of alkaloid biosynthesis in plants and plants having altered alkaloid biosynthesis**

(71) Applicant: JOHNSON & JOHNSON RESEARCH PTY LIMITED, Eveleigh, NSW 1430 (AU)
(72) Inventor: Kutchan, Toni M., 06114 Halle (Saale) (DE); Frick, Susanne, 86163 Augsburg (DE); Kemps, Katje, 09130 Chemnitz (DE)
(74) Representative: Almond-Martin, Carol

(57) **Abstract**

The invention relates to a method for altering alkaloid biosynthesis in a plant, comprising:
i) introducing into cells of a plant, an expressible exogenous nucleic acid comprising or consisting of an *(S)*-*N*-methylcoclaurine 3'-hydroxylase gene (*cyp80b*) or a derivative thereof, and
ii) optionally propagating the plant,

wherein expression of the exogenous nucleic acid in the plant or in its progeny results in altered levels of alkaloid biosynthesis.

## Description

The present invention relates to methods of modulating alkaloid biosynthesis in plants, particularly tetrahydrobenzylisoquinoline-derived alkaloids. The invention also relates to methods for producing plants having altered alkaloid biosynthesis and to methods of producing alkaloids from these plants. The invention also concerns nucleic acid molecules capable of altering alkaloid biosynthesis in plants.

Alkaloids are physiologically active, nitrogen-containing low-molecular weight compounds produced predominantly in higher plants. The tetrahydrobenzylisoquinoline-derived alkaloids include a vast number of structurally diverse molecules that vary widely in physiological activity. The muscle relaxant (+) tubocurarine, the narcotic analgesic morphine, the antitussive and analgesic codeine, and the antimicrobial berberine are examples of this group of alkaloids.

Amongst the alkaloid-producing plants, *Papaver somniferum* L. is one of the most important. It is considered to be one of the oldest cultivated medicinal plants of Europe. Opium poppy originated in the eastern Mediterranean and the ancient Sumerians used its seed for food. The "sleep-inducing" property and the medicinal value of the latex have also been known and used throughout human history (Brownstein, 1983; Husain and Sharma, 1983). *P. somniferum* contains more than eighty tetrahydrobenzylisoquinoline-derived alkaloids, including morphine, and codeine, as well as the muscle relaxant papaverine, the antitumoric agent noscapine (Ye *et al.,* 1998) and the antimicrobial sanguinarine. Although morphine biosynthesis is well understood at the enzymic level (reviewed in Kutchan, 1998), the regulation and ecological function of the morphinan alkaloids *in planta* is still unknown.

The opium poppy has been selected to produce three types of plants: ornamentals, narcotic cultivars and condiment / oilseed cultivars. The pharmaceutical and chemical uses of P. *somniferum* could be optimized and tailored to specific requirements if the alkaloid metabolism could be altered in a controlled manner. For example poppy seed oil finds use in the chemical industry for the production of pigments and lacquer, but its residual morphine levels prevents more widespread applications. Similarly, it could be desirable to optimise the production of certain alkaloids, such as thebaine, which are less likely to abuse by drug traffickers than morphine, the precursor of heroin, and yet remain useful for the production of therapeutically active derivatives.

With genetic transformation it may be possible to alter the alkaloid metabolism in commercial poppy cultivars in order to obtain varieties lacking alkaloids or to produce plants with alkaloid profiles designed for specific industrial and pharmaceutical uses. However, whilst a number of genes encoding enzymes involved in the biosynthetic pathways of the alkaloid-producing plants have now been isolated, metabolic engineering of these plants is hampered by the lack of knowledge of the regulatory mechanisms governing the pathways, including the involvement of potentially rate-limiting factors such as cytochrome P-450 and compensatory feedback mechanisms which may come into play in the plant.

Thus whilst the molecular tools now available should theoretically permit modifications to be made in the activity of enzymes acting at some steps of the alkaloid biosynthetic pathways, such changes may not necessarily lead to modification of alkaloid profiles. This is especially true if the enzymatic steps in question are upstream of a branch point in the pathway, and if changes are sought in one pathway in preference to another.

It is thus an object of the present invention to identify means of modifying alkaloid profiles in plants, particularly the tetrahydrobenzylisoquinoline-derived alkaloids. It is also an object of the invention to identify means of modifying the levels of morphinan alkaloids produced by plants such as *P.somniferum.*

The present invention meets these objectives. It has surprisingly been found that expression of the enzyme (S)-N-methylcoclaurine 3'-hydroxylase appears to limit the ability of plants such as *P.somniferum* to synthesize alkaloid, and that modulation of the expression of this enzyme has a marked effect on alkaloid production levels.

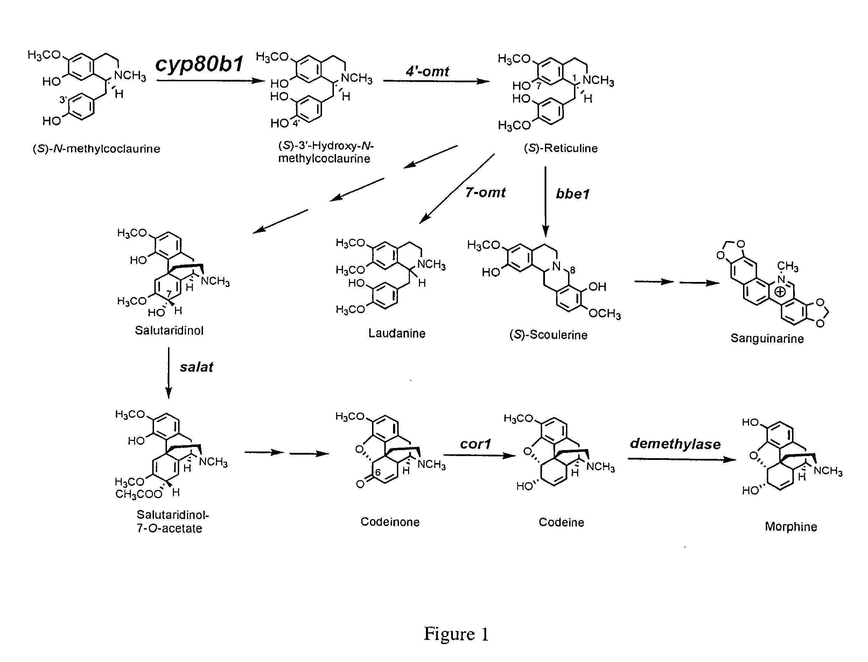

(S)-N-methylcoclaurine 3'-hydroxylase is a cytochrome P-450-dependent monooxygenase. It catalyses hydroxylation of (S)-N-methylcoclaurine to (S)-3'-hydroxy-N-methylcoclaurine on the pathway to the branchpoint isoquinoline alkaloid intermediate (S)-reticuline (Figure 1). Subsequent stereo- and regio-specific oxidation of (S)-reticuline determines which class of alkaloids will be formed, depending upon whether the morphine-, laudanine- or sanguinarine biosynthetic pathways are followed. It is thus common to these three biosynthetic pathways of P. *somniferum* alkaloids.

The cDNA of the *cyp80b1* gene encoding (S)-N-methylcoclaurine 3'-hydroxylase in *E. californica* (CYP80B1) has been isolated (Pauli and Kutchan, 1998), as has partial cDNA of a *P.somniferum* gene (Huang and Kutchan, 2000).

The present inventors have determined that up-regulation of the *cyp80b1* gene encoding (S)-N-methylcoclaurine 3'-hydroxylase leads to significantly increased total levels of alkaloids in latex. Correspondingly, down-regulation of the gene leads to reduced levels. This finding is unexpected for several reasons. First, the enzyme (S)-N-methylcoclaurine 3'-hydroxylase is cytochrome P-450-dependent, and thus the effects of its over-expression are dependent on availability of cytochrome P-450. Moreover, the metabolic step catalysed by (S)-N-methylcoclaurine 3'-hydroxylase is many steps upstream of the end products of the pathways, particularly the morphinan pathway. Since the number of downstream steps is high, the probability that one of these steps might be rate-limiting, thereby neutralising the effects of over-expression of (S)-N-methylcoclaurine 3'-hydroxylase was significant. This however does not appear to be the case. Furthermore, it could not be excluded that inhibitory feed-back mechanisms might compensate for over- or under-expression of (S)-N-methylcoclaurine 3'-hydroxylase, preventing changes in alkaloid production.

The present invention thus concerns a method for altering alkaloid biosynthesis in a plant, said method comprising modifying the expression of an *(S)-N*-methylcoclaurine 3'-hydroxylase gene in the plant. The levels of the enzyme (S)-N-methylcoclaurine 3'-hydroxylase produced by the plant are thus increased or decreased, thereby increasing or decreasing the levels of alkaloids produced.

According to the invention the modification of the expression of an *(S)-N*-methylcoclaurine 3'-hydroxylase gene can comprise induction, enhancement, suppression or inhibition of expression of endogenous (S)-N-methylcoclaurine 3'-hydroxylase gene sequences in the plant. It may also comprise modification in the spatio-temporal expression of the enzyme.

Alteration of (*S*)-*N*-methylcoclaurine 3'-hydroxylase gene expression can be achieved using any of the known techniques for gene regulation. For example, expression can be up-regulated by increasing the copy number of the endogenous gene using amplification techniques. One way of amplifying the gene is for example to insert genes encoding amplifying agents such as DHFR in the vicinity of the gene in the plant genome. Alternatively, the copy number can be increased by introducing exogenous copies of the gene using recombinant techniques. Expression can be down-regulated by use of gene-silencing techniques such as antisense suppression, sense co-suppression, RNA interference, ribozymes, DNAzymes etc. More generally, *(S)-N-*methylcoclaurine 3'-hydroxylase gene expression can be changed by modifying the regulatory sequences controlling transcription of the gene, using for example homolgous recombination to introduce or inactivate specific sequences in a targeted manner. For example, endogenous regulatory sequences can be replaced, activated, inactivated or multiplied, enabling a vast range of effects on gene expression to be achieved. A further technique allowing modification of the (*S*)-*N*-methylcoclaurine 3'-hydroxylase gene expression is mutation.

According to a preferred embodiment of the invention, the alteration of (*S*)-*N*-methylcoclaurine 3'-hydroxylase gene expression, and the concomitant alteration in alkaloid biosynthesis, is achieved by introducing into the plant, nucleic acid sequences derived from the *(S)-N-*methylcoclaurine 3'-hydroxylase gene.

More specifically, this aspect of the invention concerns a method for altering alkaloid biosynthesis in a plant, comprising :
i) introducing into cells of a plant, an expressible exogenous nucleic acid comprising or consisting of an *(S)-N*-methylcoclaurine 3'-hydroxylase gene, or a derivative thereof, and
ii) optionally propagating the plant,
wherein expression of the exogenous nucleic acid in the plant or in its progeny results in altered levels of alkaloid biosynthesis.

According to this embodiment of the invention, the exogenous nucleic acid may be any *(S)-N-*methylcoclaurine 3'-hydroxylase gene or derivative thereof.

Depending on the precise nature of the exogenous nucleic acid, its expression in the plant will give rise either to over-expression of *(S)-N*-methylcoclaurine 3'-hydroxylase, or to decreased or abolished expression of *(S)-N*-methylcoclaurine 3'-hydroxylase. It is also possible to achieve a shift in the spatial or temporal expression of the enzyme.

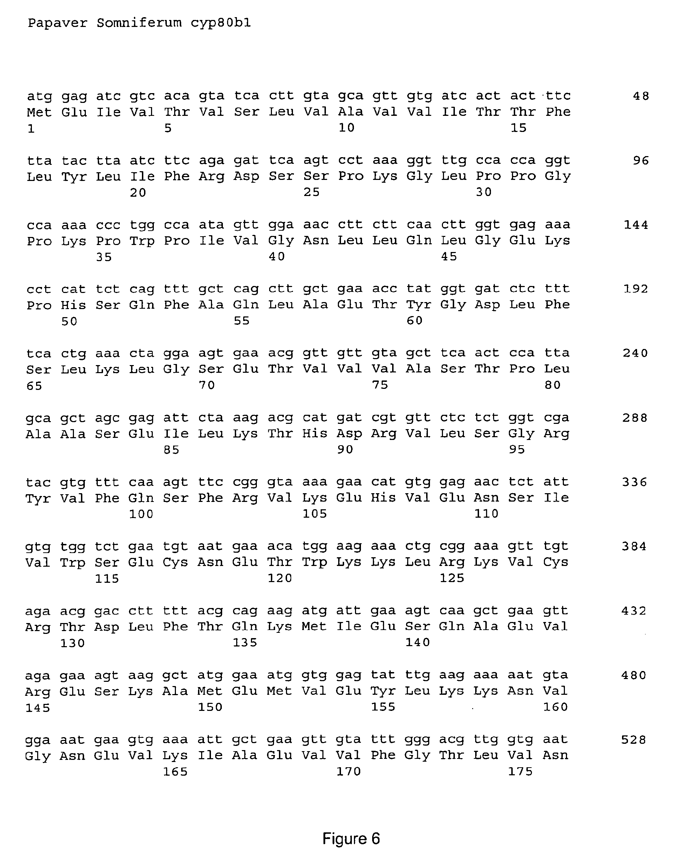

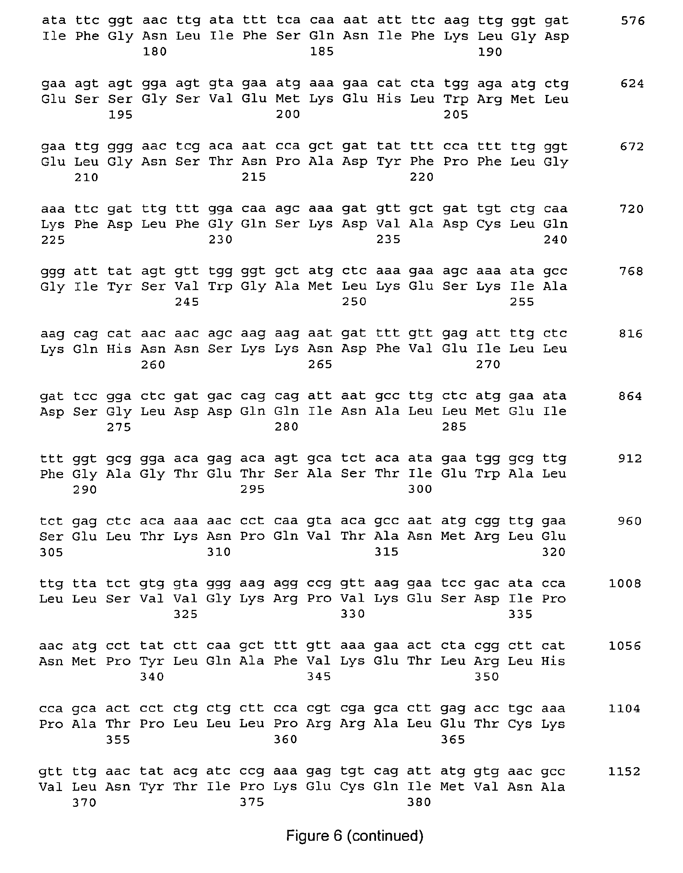

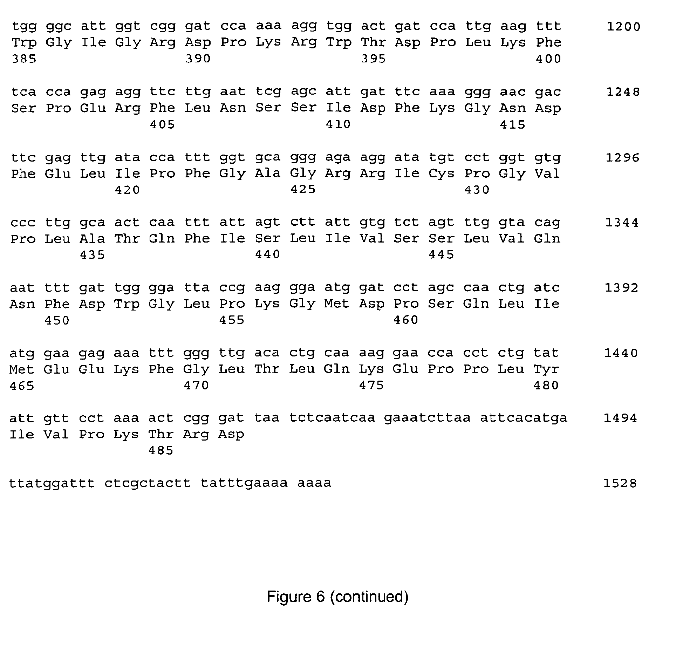

According to a first variant of the invention the exogenous nucleic acid is a *(S)-N-*methylcoclaurine 3'-hydroxylase gene, particularly a (S)-*N*-methylcoclaurine 3'-hydroxylase gene of a plant belonging to the order Ranunculales. Typical examples are the *cyp80b1* gene of *Papaver somniferum* or the *cyp80b1* gene of *Eschscholzia californica,* encoding proteins illustrated in Figures 6, 10 and 12. The genes may be the naturally occurring genes or may be altered in some way, composed for example of a coding sequence, together with heterologous transcription regulatory sequences. The coding sequences may be full length or shortened.

Use of such "sense" gene sequences as the exogenous nucleic acid of the invention gives rise, predominantly, to an over-expression of *(S)*-N-methylcoclaurine 3'-hydroxylase, and to a corresponding increase in the alkaloid levels produced by the plant. For over-expression of the enzyme using sense sequences it is preferred that the coding sequence be full length. It is also possible to obtain a co-suppression effect with the gene "sense" sequences.

The exogenous nucleic acid may also be a derivative of a (*S*)-*N*-methylcoclaurine 3'-hydroxylase gene. In this context, the term "derivative" signifies any sequence which is derived from the gene or its cDNA or its transcript, for example by fragmenting, copying, amplifying, transcribing, reverse transcribing, splicing, subjecting to polymerase, mutating, or by deleting, substituting, inserting, or chemically modifying one or more nucleotides of the gene or cDNA or transcript, or by fusing to heterologous sequences. According to the invention the derivatives have the capacity to exert a modulatory effect on the expression of the gene.

Examples of derivatives of the (*S*)-*N*-methylcoclaurine 3'-hydroxylase gene include single or double stranded sequence variants, fragments, splice variants, complementary sequences, RNA equivalents, mixed DNA/RNA equivalents, chemical analogues of said gene. Particularly preferred examples include sense and antisense sequence, ribozyme sequences, DNAzyme sequences, RNA-interference sequences, and sequences capable of giving rise to any of the foregoing or their complements.

Particularly preferred derivatives can be obtained from the *cyp80b1* gene of *Papaver somniferum.* The coding sequence of this gene is illustrated in Figure 6.

More specifically, derivatives of the *cyp80b1* gene of *Papaver somniferum* are chosen from :
i) a sequence encoding the protein sequence illustrated in Figure 6,
ii) a variant of the nucleotide sequence illustrated in Figure 6, said variant having at least 70% identity with the sequence of Figure 6 over a length of at least 1000 bases, and encoding a (S)-*N*-methylcoclaurine 3'-hydroxylase, or
iii) a fragment of sequence (i) or (ii), said fragment having a length of at least 20 nucleotides, or
iv) a sequence complementary to any one of sequences (i), (ii) or (iii), and having a length of at least 20 nucleotides, or
v) any one of sequences (i), (ii), (iii) or (iv) in double-stranded form, or
vi) the RNA equivalent of any of sequences (i), (ii), (iii), (iv) or (v), or
vii) a combination of any of the foregoing.

In accordance with the first group of derivatives (i), as defined above, the exogenous nucleic acid may comprise any sequence encoding the protein sequence illustrated in Figure 6, including all sequences arising from the degeneracy of the genetic code.

In accordance with the second group of derivatives (ii), as defined above, the exogenous nucleic acid may comprise sequence variants of the nucleotide sequence illustrated in Figure 6. Such variants may or may not code for a protein having *(S)-N*-methylcoclaurine 3'-hydroxylase activity. The sequence variant has at least 70 % identity and preferably at least 85% identity with the nucleotide sequence of Figure 6 over a length of at least 1000 bases, and differs from the sequence of Figure 6 by insertion, replacement and / or deletion of at least one nucleotide, for example at least 10 nucleotides, up to around 150 nucleotides. Examples of this type of variant include sequences corresponding to the coding sequences of *cyp80b1* genes from plant species other than *P.somniferum,* for example those of *E.californica* as illustrated in Figure 11 or Figure 13 or portions thereof.

Generally speaking the variants have the capacity to hybridise to the nucleotide sequence illustrated in Figure 6 in stringent conditions. Stringent conditions are for example those set out in Sambrook et al., Molecular Cloning : A Laboratory Manual, Cold Spring Harbor Laboratories, Cold Spring Harbor, NY, USA, 1989 pages 387-389, paragraph 11.

Particularly preferred variants have at least 99% identity with the nucleotide sequence of Figure 6 over a length of at least 1000 bases, and differ from the sequence of Figure 6 by insertion, replacement and / or deletion of at least one nucleotide, for example by one to ten nucleotides, particularly one to five nucleotides. Examples of this type of variant include allelic variants of the *cyp80b1* gene of *Papaver somniferum,* or portions thereof.

Other examples of variants which are suitable for use as exogenous nucleic acids of the invention include hybrid sequences comprising a portion of the *cyp80b1* gene of *P*. *somniferum* and a portion of a *cyp80b1* gene of a plant species other than *P.somniferum,* for example of *E.californica.* Indeed, it has been shown by the inventors that a chimeric coding sequence composed predominantly of the *P. somniferum* sequence, fused to the N-terminus of the *E.californica* sequence, is effective in modulating expression of the (*S*)-*N*-methylcoclaurine 3'-hydroxylase gene, and of altering the alkaloid profile of *P.somniferum.*

In accordance with the third group of derivatives (iii), as defined above, the exogenous nucleic acid may comprise or consist of a fragment of a sequence encoding (*S*)-*N*-methylcoclaurine 3'-hydroxylase or a fragment of a sequence variant of the (*S*)-*N*-methylcoclaurine 3'-hydroxylase gene. Such fragments have a length of at least 20 nucleotides, for example from 23 or 25 nucleotides to around 1400 nucleotides, or from 60 to 500 nucleotides. Short fragments having a length of 20 to 50 nucleotides are particularly useful in the invention for example in generating ribozymes, DNAzymes, interfering RNAs etc.

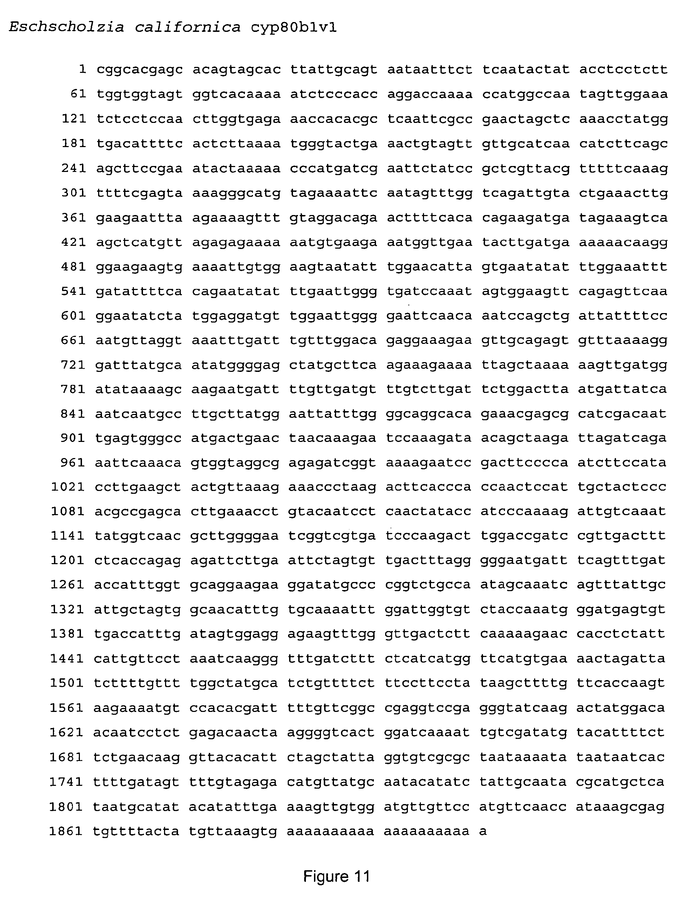

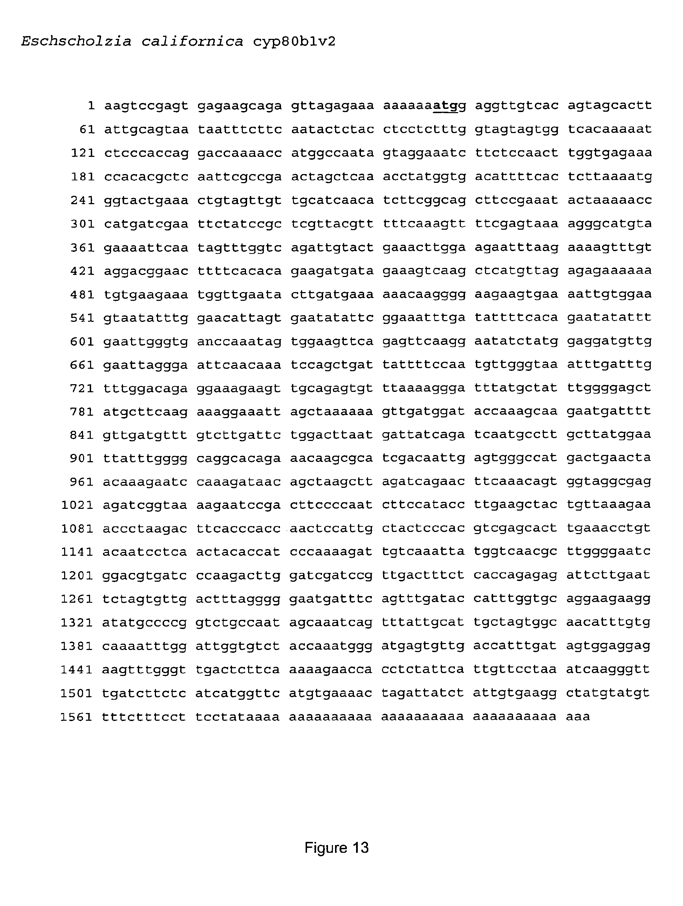

In accordance with the fourth group of derivatives (iv), as defined above, the exogenous nucleic acid may comprise or consist of a sequence which is complementary to any of the foregoing preferred derivatives. Examples of such sequences include sequences which are complementary to the nucleotide sequence illustrated in Figure 6 or to a part thereof, or to the sequence illustrated in Figure 11 or a part thereof, or to the sequence illustrated in Figure 13 or a part thereof.

In the context of the invention, "complementary" means that Watson-Crick base-pairs can form between a majority of bases in the complementary sequence and the reference sequence. Preferably, the complementarity is 100%, but one or two mismatches in a stretch of twenty or thirty bases can be tolerated. Additionally, complementary stretches may be separated by non-complementary stretches.

In accordance with a preferred embodiment of the invention the derivative comprises an antisense sequence, a ribozyme sequence, a DNAzyme sequence, an RNA-interference sequence, or a sequence capable of giving rise to any of these molecules or their complements. These sequences are generally used to down-regulate or silence expression of the (*S*)-*N-*methylcoclaurine 3'-hydroxylase gene, and to consequently give rise to reduced levels of alkaloid production in the plant.

Antisense sequences of the *(S)-N*-methylcoclaurine 3'-hydroxylase gene may correspond to the full coding sequence, i.e. from the ATG start codon to the stop codon, or may correspond to only part of the coding sequence.

Ribozymes of the invention may be directed to any part of the (*S*)-*N*-methylcoclaurine 3'-hydroxylase gene transcript, and may be single or multiple ribozymes. Hammerhead ribozymes are particularly preferred (see EP-A-0 321 201).

DNAzyme sequences i.e. sequences which comprise desoxyribonucleotide bases and have endonuclease activity may also be used according to the invention to inhibit or silence the expression of the *(S)-N*-methylcoclaurine 3'-hydroxylase gene. Such DNAZymes may be fully DNA or mixed DNA/RNA molecules, and may contain chemically modified bases (see for example International patent application WO96/17086).

Interfering RNA is a further approach which may be used according to the invention to reduce or silence the expression of the *(S)-N*-methylcoclaurine 3'-hydroxylase gene. Such interference involves generation of short double-stranded RNA molecules essentially similar to part of the nucleotide sequence of the targeted *(S)-N*-methylcoclaurine 3'-hydroxylase gene (see for example International patent application WO99/05305).

According to the invention, any of the above derivatives may comprise at least one nucleotide analogue in replacement of, or in addition to, a naturally occurring nucleotide. Ribonucleotide and deoxyribonucleotide derivatives or modifications are well known in the art, and are described, for example, in Principles of Nucleic Acid Structure (Ed, Wolfram Sanger, Springer-Verlag, New York, 1984), particularly pages 159-200), and in the CRC Handbook of Biochemistry (Second edition, Ed, H. Sober, 1970). A large number of modified bases are found in nature, and a wide range of modified bases have been synthetically produced. For example, amino groups and ring nitrogens may be alkylated, such as alkylation of ring nitrogen atoms or carbon atoms such as N1 and N7 of guanine and C5 of cytosine; substitution of keto by thioketo groups; saturation of carbon=carbon double bonds. Bases may be substituted with various groups, such as halogen, hydroxy, amine, alkyl, azido, nitro, phenyl and the like. Examples of suitable nucleotide analogues are listed in Table I below. In accordance with this embodiment of the invention, synthetic genes comprising one or more nucleotide analogues, for example methylated bases, are made, for example by chemical synthesis, and can be introduced into cells for a transient expression process in vivo.

**Table I : Nucleotide Analogues**

| **Abbreviation** | **Description** |
|---|---|
| ac4c | 4-acetylcytidine |
| chm5u | 5-(carboxyhydroxylmethyl)uridine |
| cm | 2'-O-methylcytidine |
| cmnm5s2u | 5-carboxymethylaminomethyl thiouridine |
| d | dihydrouridine |
| fm | 2'-O-methylpseudouridine |
| galq | β, D-galactosylqueosine |
| gm | 2'-O-methylguanosine |
| I | inosine |
| i6a | N6-isopentenyladenosine |
| m1a | 1-methyladenosine |
| m1f | 1-methylpseudouridine |
| m1g | 1-methy[guanosine |
| ml1 | 1-methylinosine |
| m22g | 2,2-dimethylguanosine |
| m2a | 2-methyladenosine |
| m2g | 2-methylguanosine |
| m3c | 3-methylcytidine |
| m5c | 5-methylcytidine |
| m6a | N6-methyladenosine |
| m7g | 7-methylguanosine |
| mam5u | 5-methylaminomethyluridine |
| mam5s2u | 5-methoxyaminomethyl-2-thiouridine |
| manq | β, D-mannosylmethyluridine |
| mcm5s2u | 5-methoxycarbonylmethyluridine |
| mo5u | 5-methoxyuridine |
| ms2i6a | 2-methylthio-N6-isopentenyladenosine |
| ms2t6a | N-((9-β-ribofuranosyl-2-methylthiopurine-6-yl)carbamoyl)threonine |
| mt6a | N-((9-[β-ribofuranosylpurine-6-yl)N-methyl-carbamoyl)threonine |
| mv | uridine-5-oxyacetic acid methylester |
| o5u | uridine-5-oxyacetic acid (v) |
| osyw | wybutoxosine |
| p | pseudouridine |
| q | queosine |
| s2c | 2-thiocytidine |
| s2t | 5-methyl-2-thiouridine |
| s2u | 2-thiouridine |
| s4u | 4-thiouridine |
| t | 5-methyluridine |
| t6a | N-((9-β-D-ribofuranosylpurine-6-yl)carbamoyl)threoninetm 2'-O-methyl-5-methyluridine |
| um | 2'-O-methyluridine |
| yw | wybutosine |
| x | 3-(3-amino-3-carboxypropyl)uridine, (acp3)u |
| araU | β, D-arabinosyl |
| araT | β, D-arabinosyl |

In accordance with the invention, the plant whose alkaloid production is altered is a plant which naturally has the capacity to produce endogenous benzylisoquinoline-derived alkaloids. Such plants contain at least one copy of an endogenous *(S)-N-*methylcoclaurine 3'-hydroxylase gene. Plants belonging to the order Ranunculales are examples of such plants, particularly plants belonging to any one of the families Papaveraceae, Euphorbiaceae, Berberidaceae, Fumariaceae or Ranunculaceae. A particularly preferred plant belongs to the genus Papaver, for example plants of the species *Papaver somniferum, Papaver bracteatum, Papaver setigerum, Papaver orientale, Papaver pseud-orientale, Papaver cylindricum,* or *Papaver rhoeas.*

*Papaver somniferum* is the most preferred plant on account of its capacity to produce morphinan alkaloids.

In the context of the invention, the alteration of alkaloid biosynthesis particularly relates to the alteration of tetrahydrobenzylisoquinoline-derived alkaloids, including the alkaloids found in latex such as morphine, codeine, codeinone, thebaine, oripavine, reticuline, (S)-laudanine and laudanosine. Implementation of the methods of the invention affects levels of alkaloids of both the morphine and laudanine biosynthetic pathways.

In particular, the altered alkaloid biosynthesis affects levels of at least one alkaloid selected from the group consisting of morphine, codeine, codeinone, thebaine, oripavine, reticuline, (S)-laudanine and laudanosine. In plants of Papaver the alteration may for example result in an increase or a decrease in the amount of total alkaloid in latex of the plant. Changes involving increases or decreases in as much as four or five times the total amount of alkaloid have been obtained. Such changes have been observed in normal commercial varieties of *P.somniferum* and also in elite varieties which already have high alkaloid production levels. The proportions of the individual alkaloids in the latex of the plant may or may not be altered. The method of the invention can thus be used to generate high morphine lines, low morphine lines, high thebaine lines, high codeine lines etc.

Particularly preferred examples result in an increase in the proportion of the morphine levels in the latex of the plant.

Preferred variants for increasing alkaloid production in *P.somniferum* invove the use of a sequence comprising or consisting of a "sense" sequence encoding the *P.somniferum* CYP80B1 protein illustrated in Figure 6 as exogenous nucleic acid of the invention

Preferred variants for decreasing alkaloid production in *P.somniferum* invove the use of a sequence comprising or consisting of an "antisense" sequence encoding the *P.somniferum* CYP80B1 protein illustrated in Figure 6 as exogenous nucleic acid of the invention

According to the invention, the exogenous nucleic acids are introduced into plant cells using transfection or transformation techniques conventional in the art, in conditions allowing expression of the exogenous nucleic acids. A number of transformation techniques have been reported for Papaver. For example, microprojectile bombardment of cell suspension cultures may be used. Transformation may also be effected using Agrobacterium, particularly *Agrobacterium tumefaciens* (see for example WO 99/34663), *or Agrobacterium rhizogenes,* using either cell suspension cultures or tissue explants. A number of further techniques are available and are known to the skilled man.

With regard to regeneration of the plants, techniques for recovering whole *P*. *somniferum* plants from tissue cultures are now known. Opium poppy has been regenerated via somatic embryogenesis (Nessler, 1982; Schuchmann and Wellmann, 1983; Yoshikawa and Furuya, 1983; Wakhlu and Bajwa, 1986; Ovecka *et al.,* 1996; Kassem and Jacquin, 2001; Chitty *et al.,* 2003), via anther culture and microspore-derived calluses and plants (Dieu *et al.,* 1988), and via shoot organogenesis (Park and Facchini, 2000 a).

The invention also relates to a method for producing a plant having altered alkaloid biosynthesis, comprising
i) modifying the expression of an *(S)-N-*methylcoclaurine 3'-hydroxylase gene in the plant by a method according to any one of claims 1 to 38, and
ii) optionally propagating the plant.

The propagation of the plant may be effected by means of self-fertilisation or cross-fertilisation, followed by selection of progeny plants having the capacity to exhibit altered alkaloid biosynthesis. Such plants may exhibit a phenotypically altered alkaloid production, or alternatively may only exhibit such a phenotypic trait on further propagation, depending upon whether the plant is homozygous or heterozygous for the change, and on whether the trait is dominant or recessive.

The propagation of the plant may also be effected by means of clonal propagation.

The invention also relates to methods for producing alkaloids comprising :
- producing a plant having altered alkaloid biosynthesis in accordance with any of the methods described herein,
- harvesting the plant parts containing the alkaloids,
- extracting the alkaloids from the plant parts.

The plant parts which are harvested are usually poppy capsules or straw or latex, although some species for example *P.bracteatum* contains alkaloids in the roots. Extraction is carried out in accordance with conventional techniques.

The invention further relates to the plants, particularly *P.somniferum* having altered alkaloid biosynthesis which can be obtained by the methods of the invention. Such plants contain in their genetic material, an exogenous nucleic acid comprising or consisting of an (*S*)-*N-*methylcoclaurine 3'-hydroxylase gene, or a derivative thereof. The plants can be distinguished from naturally occurring plants in that they either contain the (S)-N-methylcoclaurine 3'-hydroxylase gene at a copy number which is higher than that occurring naturally, or they contain an (*S*)-*N*-methylcoclaurine 3'-hydroxylase gene in a genetic location which is different from that occurring naturally, or the exogenous nucleic acid is heterologous with respect to the genome of the plant. "Heterologous" in this context means that the *(S)-N*-methylcoclaurine 3'-hydroxylase gene is not identical to the naturally occurring gene, for example it comprises heterologous transcription regulatory signals. The regulatory signals used in the gene preferable permit expression of the exogenous nucleic acid in the vascular bundle of the plant.

Depending on the method used for introducing the exogenic nucleic acid, it may be stably integrated into the genome of the plant, or alternatively may be on an autonomously replicating virus or plasmid.

The invention also relates to plant cells, for example cell cultures or cell lines, containing in their genetic material, an exogenous nucleic acid comprising or consisting of an *(S)-N-*methylcoclaurine 3'-hydroxylase gene, or a derivative thereof.

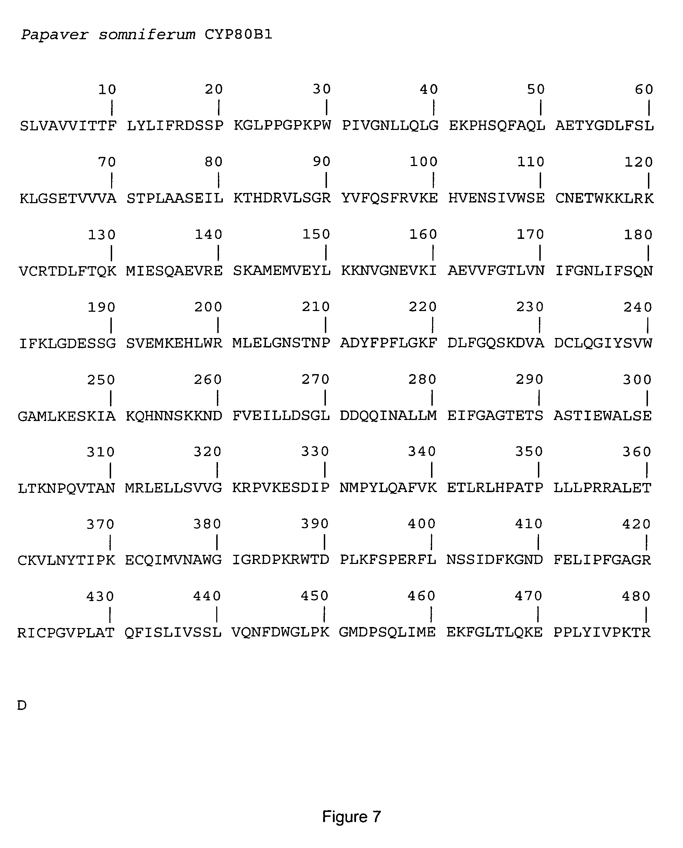

The invention also relates to a nucleic acid encoding a *(S)*-*N*-methylcoclaurine 3'-hydroxylase, said nucleic acid comprising the *P.somniferum cyp80b1* coding sequence illustrated in Figure 7.

The invention also relates to a hybrid nucleic acid encoding a *(S)*-*N*-methylcoclaurine 3'-hydroxylase comprising a portion of the *P.somniferum cyp80b1* coding sequence illustrated in Figure 7 and a portion of the E.californica *cyp80b1* coding sequence illustrated in Figure 11 or Figure 13.

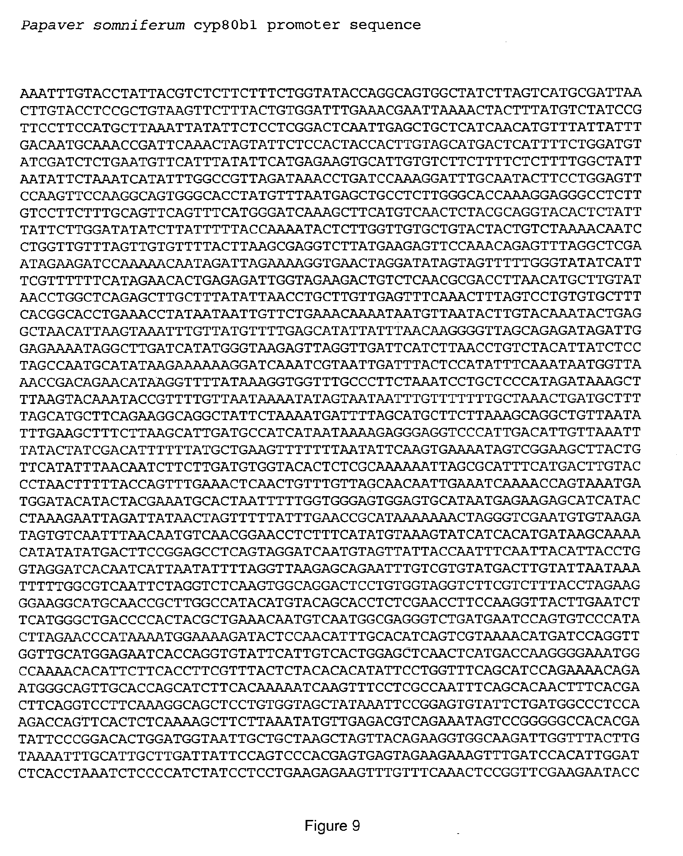

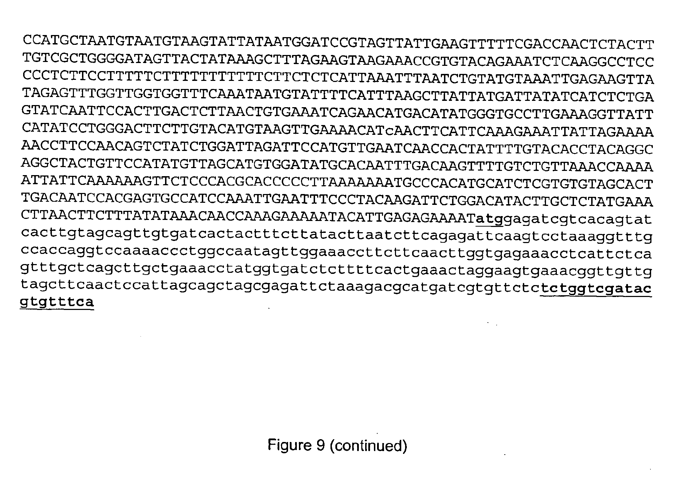

The invention also relates to a nucleic acid comprising or consisting of the 5' regulatory sequences of the *P.somniferum cyp80b1* gene illustrated in Figure 9. This sequence, or a functional part thereof, can be used in chimeric genes for initiation and / or control of transcription of genes in plants. It is particularly advantageous to use this promoter sequence for expression of heterologous genes *in P.somniferum* and other plants of the Papver genus.

Further aspects of the invention are illustrated in the Figures.

### Figure Legends

**Figure 1**. Schematic presentation of the biosynthetic pathway from (S)-norcoclaurine to codeine, laudanine and (S)-scoulerine in *P. somniferum.* The position of the cDNA *cyp80b1* used in this work is indicated in large print.

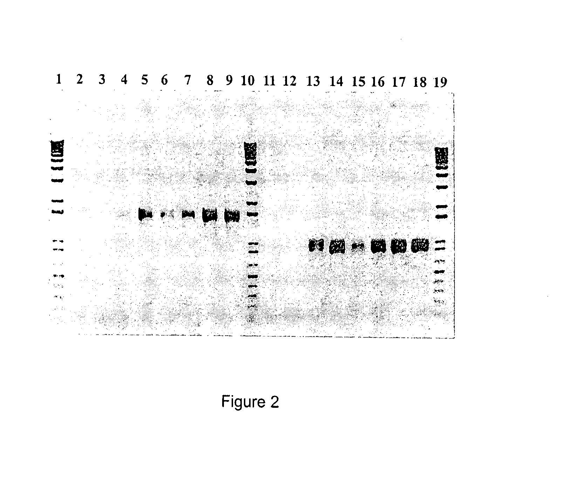

**Figure 2.** Polymerase chain reaction amplification of transgene from genomic DNA from wild type (WT) and transgenic plants of the T₃ generation. Reaction mixtures were resolved by agarose gel electrophoresis and DNA was visualized with ethidium bromide. Lane 1) 1 kb+ molecular size marker; 2) negative control (water instead of DNA template); 3) negative control (genomic DNA from WT); 4) genomic DNA template from T₃-15.7/2; 5) genomic DNA template from T₃-15.7/13; 6) genomic DNA template from T₃-15.7/14; 7) genomic DNA template from T₃₋17.6/10; 8) *sense-cyp80b1* pPLEX X002; 9) *antisense-cyp80b1* pPLEX X002; 10) 1kb+ molecular size marker; 11) negative control (water instead of DNA template); 12) negative control (genomic DNA from WT); 13) genomic DNA template from T₃-15.7/2; 14) genomic DNA template from T₃-15.7/13; 15) genomic DNA template from T₃-15.7/14; 16) genomic DNA template from T₃-17.6/10; 17) *sense-cyp80b1* pPLEX X002; 18) *antisense-cyp80b1* pPLEX X002; 19) 1kb+ molecular size marker. Lanes 2 - 9 are results from an amplification of the *cyp80b1* transgene using oligodeoxynucleotide primers S4S4 and Me1'; lanes 11 - 18 are results from an amplification of a fragment of the *nptll* gene using oligodeoxynucleotide primers NPTIIs and NPTIIas. The primer sequences are given in the Material and Methods.

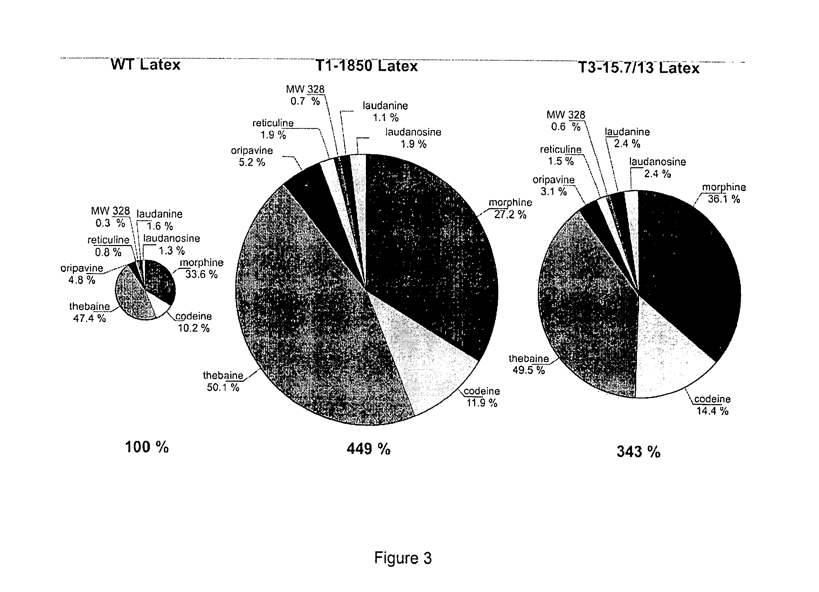

**Figure 3.** Comparative alkaloid analysis of latex from *P. somniferum* wild type (WT), a T₁ generation plant (T1-1850) that resulted from transformation with *sense-cyp80b1* and a T₃ generation plant (T3-15.7/13) that resulted from transformation with *sense-cyp80b1.* WT values represent the average alkaloid content of 29 wild type plants. Alkaloids in latex were calculated as µg alkaloid / 100 µg soluble protein, then normalized to 100% to produce the individual divisions within pie graphs, representing relative ratios of the latex alkaloids. Total WT alkaloids were set to 100% to produce the relative diameters of the pie graphs, representing the relative total alkaloid contents of the transgenic compared to the WT.

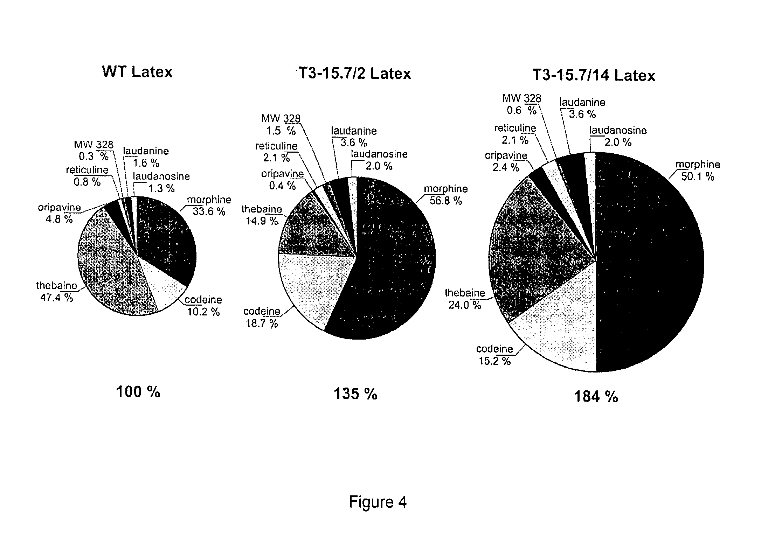

**Figure 4.** Comparative alkaloid analysis of latex from *P. somniferum* wild type (WT) and two T₃ generation plants (T3-15.7/2 and T3-15.7/14) that resulted from transformation with *sense-cyp80b1.* WT values represent the average alkaloid content of 29 wild type plants. Alkaloids in latex were calculated as µg alkaloid / 100 µg soluble protein, then normalized to 100% to produce the individual divisions within pie graphs, representing relative ratios of the latex alkaloids. Total WT alkaloids were set to 100% to produce the relative diameters of the pie graphs, representing the relative total alkaloid contents of the transgenic compared to the WT.

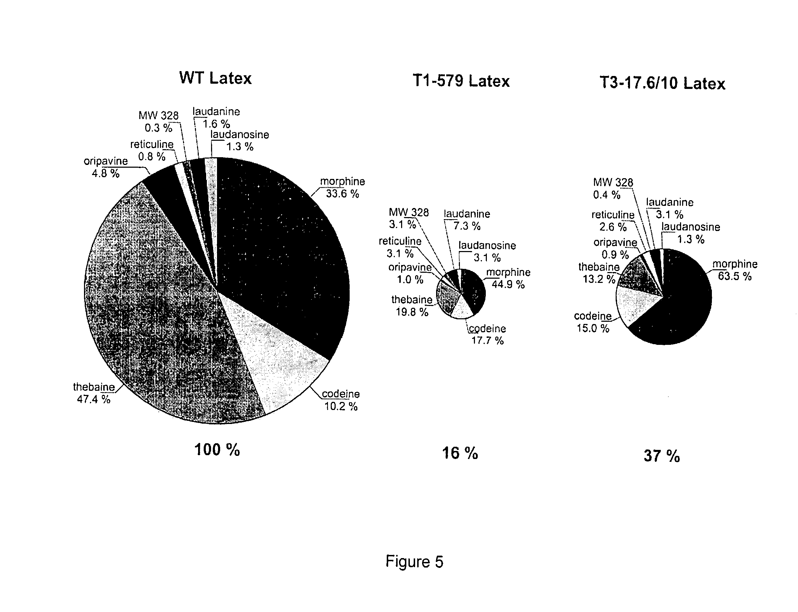

**Figure 5.** Comparative alkaloid analysis of latex from *P. somniferum* wild type (WT), a T₁ generation plant (T1-579) that resulted from transformation with *antisense-cyp80b1* and a T₃ generation plant (T3-17.6/10) that resulted from transformation with *antisense-cyp80b1.* WT values represent the average alkaloid content of 29 wild type plants, representing relative ratios of the latex alkaloids. Alkaloids in latex were calculated as µg alkaloid / 100 µg soluble protein, then normalized to 100% to produce the individual divisions within pie graphs. Total WT alkaloids were set to 100% to produce the relative diameters of the pie graphs, representing the relative total alkaloid contents of the transgenic compared to the WT.
**Figure 6**. *Papaver somniferum cyp80b1coding* sequence and amino acid sequence. Start and stop codons are indicated.
**Figure 7**. *Papaver somniferum* CYP80B1 partial amino acid sequence. The first five amino acids and methionine are missing.

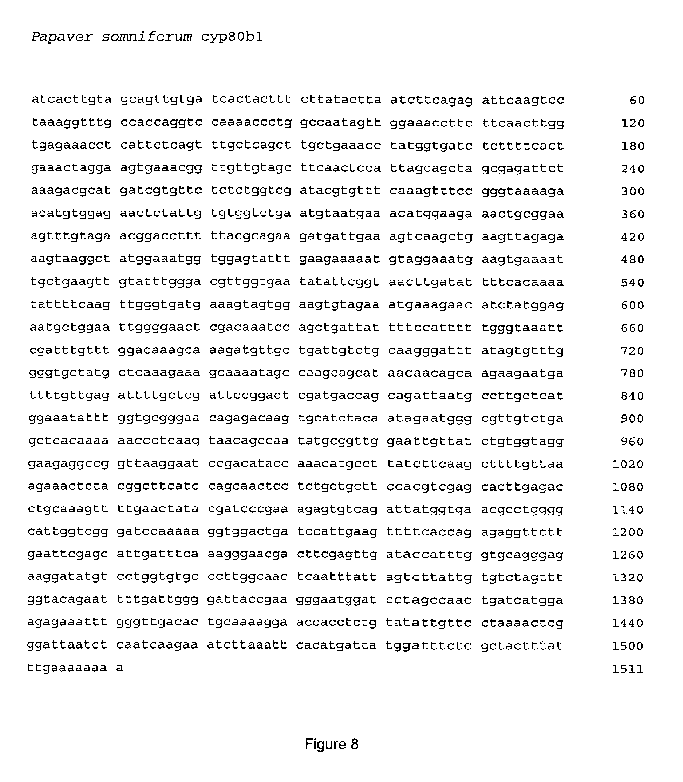

**Figure 8**. *Papaver somniferum cyp80b1* partial cDNA sequence, coding for the amino acid sequence of Figure 7. The first 17 nucleotides within the coding sequence are missing. The TAA stop codon is underlined.
**Figure 9.** *Papaver somniferum cyp80b1* promoter sequence, together with a 5' portion of the coding sequence. ATG start codon is indicated. The position of the PCR primer used for genome walking to generate the 5'non-coding region is also indicated.

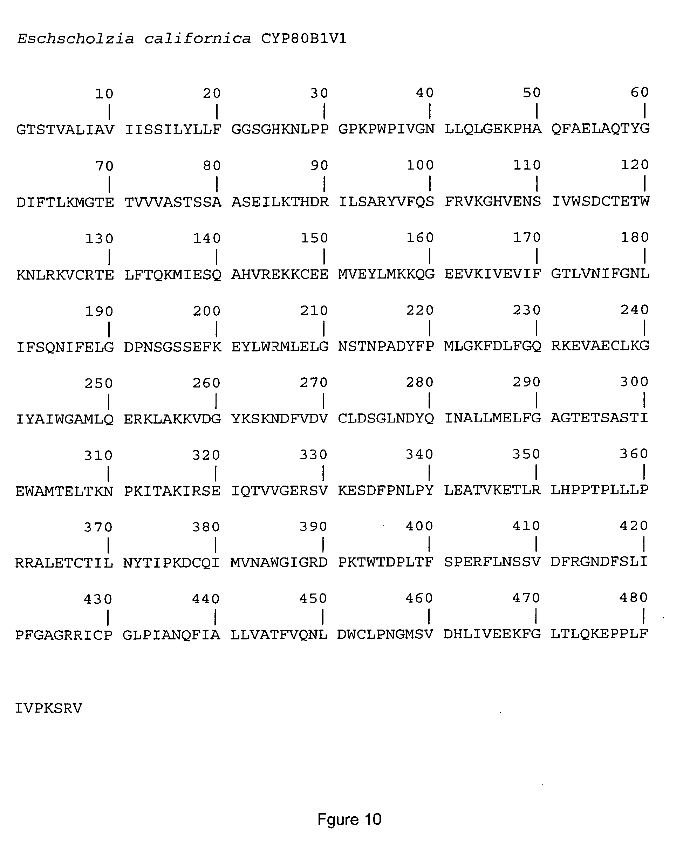

**Figure 10.** *Eschscholzia californica* CYP80B1V1 partial amino acid sequence. The first five amino acids are missing.
**Figure 11**. *Eschscholzia californica cyp80b1v1* partial cDNA sequence, coding for the amino acid sequence of Figure 10.

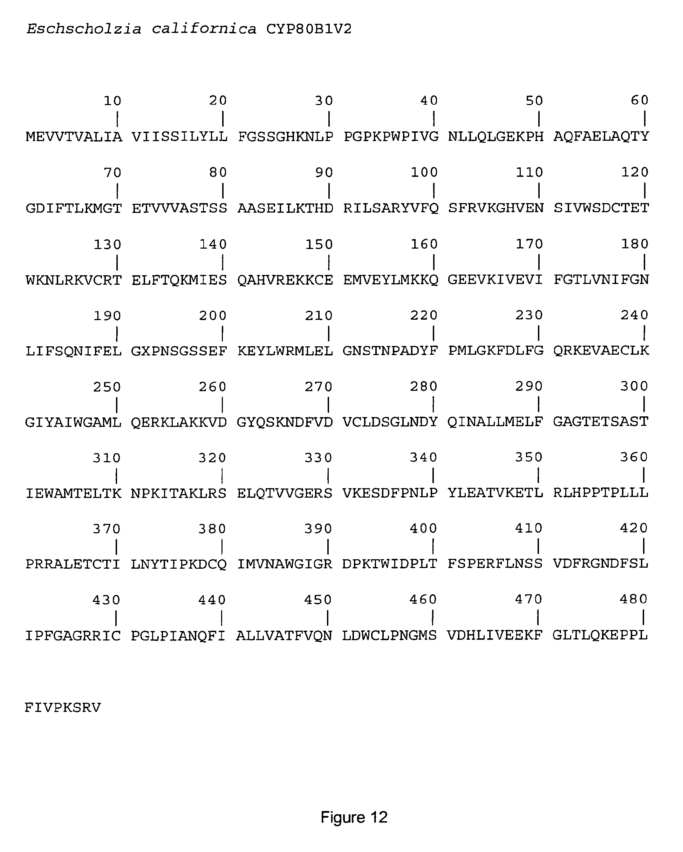

**Figure 12.** *Eschscholzia californica* CYP80B1V2 complete amino acid sequence.
**Figure 13.** *Eschscholzia californica cyp80b1v2* cDNA sequence, coding for the amino acid sequence of Figure 12.

### EXAMPLES

The following examples describe the A. tumefaciens-mediated transformation and subsequent regeneration of opium poppy with introduction of an antisense- or sense-expressed (S)-*N-*methylcoclaurine 3'-hydroxylase encoding cDNA *cyp80b1* into seedling explants. The transgene altered alkaloid levels in latex; *sense-cyp80b1* expressing plants showed increased levels of alkaloids and *antisense-cyp80b1* expressing plants showed reduced levels of alkaloids in latex.

### Example 1 : Material and Methods

### 1.1 Construction of transformation vectors

Recombinant *cyp80b1* from *P*. *somniferum* L. was cloned into pUC-18 vector (Pauli and Kutchan, 1998; Huang and Kutchan, 2000). The *cyp80b1* was a chimeric sequence composed of the partial *P*. *somniferum* coding sequence illustrated in Figure 8, completed at the 5' end by nucleotides from the *Eschscholzia califorica* cyp80b1v2 sequence illustrated in Figure 13, encoding the first five amino terminal amino acids and methionine (ATG GAG GTT GTC ACA GTA). The *Bg*lII/XhoI restriction fragment was resolved by agarose gel electrophoresis, purified with the Wizard® PCR Preps DNA Purification System (Promega) and blunted with Pfu-polymerase for 30 min at 75°C. After a second purification with the Wizard® System, the blunted DNA fragment was ligated into the *Sna*BI digested vector pPLEX X002 (vector from Phil Larkin, CSIRO Plant Industry, Canberra).

The plasmids *antisense-cyp80b1* and *sense-cyp80b1* pPLEX X002 were transformed by heat shock (30 sec, 42°C) in competent *E*. coli XL1-BlueMRF'. Positive transformants were plated onto Luria-Bertani medium (Maniatis *et al.,* 1982) containing 100 mg/l ampicillin or 50 mg/l spectinomycin. Positive colonies growing on LB-spectinomycin but not on LB-ampicillin were used to inoculate an over night culture in LB medium containing 50 mg/l spectinomycin. The binary plasmids were isolated from 3 ml overnight cultures according to the method of Birnboim and Dolly (1979). The bacterial suspensions were centrifuged (5 min, 10000 rpm) and the pellets were resuspended in 200 µl GTE buffer (50 mM glucose, 25 mM Tris/HCl pH 8.0, 10 mM EDTA pH 8.0, 100 µg/ml RNAse A). The lysis of the cells occurred by adding 300 µl 0.2 M NaOH / 1% (w/v) SDS to the solution.

After precipitation of proteins with 300 µl 3 M potassium acetate pH 4.8, the suspensions were incubated on ice for 10 min, centrifuged (10 min, 14000 rpm) and the clear supernatants were then transferred into new microcentrifuge tubes. Plasmid DNA was precipitated with 1 volume 100% isopropanol and pelleted by centrifugation (10 min, 14000 rpm). After washing with 500 µl 70% (v/v) ethanol, the pellet was dried 15 min at room temperature and resolved in 20 µl 0.1x TE buffer. The direction of the inserted *cyp80b1* cDNA was confirmed by restriction enzyme digest with *P*acl/*P*vul or *Pvul* prior to nucleotide sequence determination with ABI PRISM™ BigDye Terminator Cycle Sequencing Kit according to the manufacturer's instructions.

### 1.2 Preparation of A. tumefaciens

The binary vectors *antisense-cyp80b1* and *sense-cyp80b1* pPLEX X002 were transformed by electroporation (BioRad Gene Pulser, volts: 2,0 kV; resistance: 400 Ω; capacitance 25 µF) into the disarmed A. *tumefaciens* strain AGL1 (Lazo *et al.,* 1991) and plated onto LB medium containing 50 mg/l spectinomycin, 20 mg/l rifampicin and 50 mg/l carbenicillin. Transformed A. *tumefaciens* cultures were grown to mid-log phase (A₆₀₀ = 0.5) at 28°C on a gyratory shaker at 220 rpm in liquid LB medium containing 50 mg/l spectinomycin. The bacterial cells were collected by centrifugation for 10 min at 8000 rpm and resuspended at a cell density of A₆₀₀ = 0.3 in liquid LB medium. The solutions were used directly for the transformation of *P. somniferum* explants.

### 1.3 Seed sterilization and germination

The genotype of *P. somniferum* used in this study was the industrial inbreed line C048-6-14-64 obtained from Tasmanian Alkaloids Pty Ltd, Westbury. Seeds were surface-sterilized by washing for 1 min with 70% (v/v) ethanol and then in 0.8% (v/v) sodium hypochlorite solution plus 1-2 drops of autoclaved Triton-X-100 for 25 min with agitation, then rinsed five times in sterile distilled water or until the hypochlorite sent is removed. Seeds were blotted dry on sterile filter paper and 37 seeds were placed in 100 x 20 mm Petri dishes containing B5O medium, which consists of B5 macronutrients, micronutrients, iron salts and vitamins (Gamborg *et al.,* 1968), 20 g/l sucrose, 2 g/l MES using 0.8% (w/v) Sigma agar as the gelling agent (Larkin *et al.,* 1999; Chitty *et al.,* 2003). pH was adjusted with 1M NaOH to 5.6 (Larkin *et al.,* 1999; Chitty *et al.,* 2003) and then sterilized by autoclaving at 121 °C for 20 min. Dishes were sealed with Micropore™ surgical tape (3M Pharmaceuticals) and stored at 4°C for 24 h. Seeds were germinated in a growth chamber at 23°C under standard daylight (OSRAM L36 W/72-965 BIOLUX, München, Germany) with a flux rate of 160 µMol/m²s, 0.5 m/s air circulation and a 16-h photoperiod.

### 1.4 Transformation, selection and regeneration of transgenic plants

Transgenic plants have been regenerated according to Larkin *et al.* (1999) and Chitty *et al.* (2003). Hypocotyls from 8-day-old seedlings were isolated by removing the root and the cotyledons bellow the cotyledonary node. The explants were immediately inoculated by immersion in a liquid *A. tumefaciens* culture for 15 min. Control explants were incubated in sterile distilled water. The hypocotyls were transferred directly to 19D medium. Callusing medium (CM) also referred to as 19D is identical to B5O except that it includes 1 mg/l 2,4-D (2,4-dichlorophenoxy acetic acid) (Larkin *et al*., 1999; Chitty *et al.,* 2003). After four to five days of co-cultivation, the explants were washed in sterile, distilled water until the water was clear of *Agrobacterium,* blotted dry on sterile filter paper and transferred directly to19D medium containing 150 mg/l timentin (GlaxoSmithKline, Worthing; Duchefa Biochemie BV, Haarlem) and 25 mg/l paromomycin. Control explants were incubated on 19D medium. Four months after transformation, embryogenic callus referred to as type II (Larkin *et al*, 1999; Chitty *et al*., 2003) developed and was transferred to B50 medium lacking growth regulators containing 150 mg/l timentin and 25 mg/l paromomycin. Control calli were incubated on B5O medium without antibiotics. All cultures were transferred after 21 days to fresh medium and were grown in a growth chamber at 23°C under standard daylight as described above. After eight weeks on B5O medium, fully differentiated somatic embryos developed and were grown to plantlets in tissue culture, then after sufficient shoot and root growth, the plants were transferred to soil (80% compost, 20% perlite). The transgenic as well as control plants were grown in a green house under a High Pressure Sodium Lighting System (Philips, SON-T AGRO 400W) at 20-24°C with a 18-20°C temperature shift at night, a relative humidity of 60% and a 16-h photoperiod. The opium poppy plants regenerated directly from a callus were designated as the T₀ generation and their self-pollinated progeny as the T₁, T₂ and T₃ generations.

### 1.5 Harvest of leaves, roots and latex

When the plants reached a size of at least 20 cm, leaves were collected and used for the isolation of DNA and RNA. The poppy flower was self-pollinated on the day the petals opened. Exactly two days after the petals dropped, latex was collected by incising the capsule longitudinally with a scalpel. The exuded latex was collected with a pipette and was stored in 200 µl collection buffer (100 mM potassium phosphate pH 7.2, 500 mM D-mannitol, 20 mM ascorbic acid) (Decker *et al.,* 2000) and frozen in liquid nitrogen. The samples were stored at - 80°C prior to analysis. After ripening of the capsule, the seeds were collected and after determination of root weight, the roots were frozen in liquid nitrogen and were stored at -80°C prior to analysis.

### 1.6 Nucleic acid isolation

Plant genomic DNA and total RNA was extracted with modified standard protocols as described in Maniatis *et al.* (1982) and Cathala *et al.* (1983). All steps if not otherwise mentioned were carried out at room temperature. 1 g leaf material was ground in a mortar with liquid nitrogen. The tissue was mixed with 3.5 ml lysis buffer (10 mM Tris/HCl pH 7.5, 50 mM NaCl, 1% [w/v] SDS, 4% [w/v] PVPP, 1 mM EDTA pH 8.0, 14 mM β-mercaptoethanol) and 3.5 ml phenolchloroform-isoamylalcohol (25:24:1) and extracted for 30 min on a gyratory shaker. After 10 min centrifugation (5000 rpm) the aqueous layer was first extracted with 3.5 ml phenol-chloroform-isoamylalcohol and after a centrifugation as described above extracted with 3 ml chloroform. The phases were separated by centrifugation (10 min, 5000 rpm) and the aqueous layer was then mixed with 0.1 volume 3 M sodium acetate pH 5.2, 1 volume isopropanol and incubated at - 20°C for 1 h. After centrifugation (20 min, 5000 rpm) the pellet was washed with 70% (v/v) ethanol, dried at 37°C and was resuspended in 300 µl TE (7.5 mM Tris/HCl pH 8.0, 0.75 mM EDTA) buffer. The total RNA was precipitated over night at 4°C with 300 µl 6 M LiCl. On the next morning the solution was centrifuged for 15 min with 14000 rpm at 4°C. The RNA pellet was washed with 500µl 70% (v/v) ethanol, dried at 37°C and resuspended in 50 µl TE buffer. The DNA containing supernatant was precipitated with 0.1 volume 3 M sodium acetate pH 5.2, 1 volume isopropanol and incubated at -20°C for 20 min. After centrifugation (15 min, 14000 rpm, 4°C) the pellet was washed with 500 µl 70% (v/v) ethanol, dried at 37°C and resuspended in 50 µl TE buffer.

### 1.7 Polymerase chain reaction analysis of transformation

PCR was used to test the transformation of transgenic plants. DNA amplification was performed under the following conditions: a) for *nptll:* Primer sequences, NPTlls: 5'-GAG GCT ATT CGG CTA TGA CTG -3'and NPTllas: 5'- ATC GGG AGC GGC GAT ACC GTA -3' (Bonhomme *et al.,* 2000); cycle , 94°C, 3 min, 35 cycles of 94°C, 30 sec; 60°C, 30 sec; 72°C, 1 min, cycle 72°C, 5 min. At the end of all cycles the reaction was cooled to 4°C; b) for *cyp80b1* (S4S4 promoter and Me1' terminator): Primer sequences, S4S4: 5'- TAA GCG TAC TCA GTA CGC TTC -3' and Me1': 5'- GCA TTA CAA CAT GCA TCT GAC-3'; cycle, 94°C, 3 min, 35 cycles of 94°C, 1 min; 55°C, 1 min; 72°C, 2 min, cycle 72°C, 5 min. At the end of all cycles, the reaction was cooled to 4°C. The amplified DNA was resolved by agarose gel electrophoresis using standard protocols.

### 1.8 HPLC analysis of benzylisoquinoline alkaloids

Latex samples of wild type or transgenic plants were thawed on ice and treated 6 min in a ultrasound waterbath at 4°C. The sample was centrifuged 30 min with 12500 rpm at 4°C. The supernatant was used to determine the protein concentration. An aliquot of the supernatant was mixed with an internal standard (dihydrocodeinone), diluted with 70% (v/v) ethanol and centrifuged. The pellet, to which an internal standard was also added, was treated under sonification with a defined volume of 70% (v/v) ethanol and was separated on a reversed phase column. Samples were analysed by HPLC on a liquid chromatography system (Agilent Technologies, Waldbronn, Germany) using a reverse phase column (LiChrospher 60 RP-select B, 4 x 250 mm, 5µm, UV detection at 210, 282 and 440 nm). The alkaloids were separated at a flow rate 1 ml min⁻¹ and using the gradient H₂O: acetonitrile (0-25 min : 0-46% acetonitrile, 25-26 min 46-100% acetonitrile, 26-33 min 100% acetonitrile) containing 0.01% (v/v) phosphoric acid. Peaks have been routinely identified from their UV spectra and by comparison of their retention times to those of authentic standards. Subsequently the identity of the peaks was confirmed by liquid chromatography-mass spectroscopy.

Root samples were homogenized with 10 ml 70% (v/v) ethanol and 20 µg dihydrocodeinone as internal standard. The filtrate was evaporated, made basic with sodium bicarbonate and subsequently extracted with ethyl acetate. The concentrated samples were dissolved in 500 µl 70% (v/v) ethanol and an aliquot was analysed by HPLC using the equipment as described above with a modified gradient as follows: 0-25 min : 0-60% acetonitrile, 25-26 min 100% acetonitrile, 26-33 min 100% acetonitrile and UV detection at 282 nm.

### 1.9 Liquid Chromatography-Mass Spectroscopy

The positive electrospray ionisation (ESI) mass spectra were obtained with a Mariner-TOF 5232 instrument (Applied Biosystems, Weiterstadt, Germany) using a turbulon spray (spray tip potential 5500V, SCIEX heater 310°C, nozzle potential 160 V, nebulizer and auxillary gas (nitrogen) coupled to a modified Agilent 1100-LC equipped with a Lichrospher 60 RP-select B column (2x125 mm i.d.; 5µm)). The analysis was performed with a gradient system consisting of H₂O - CH₃CN (each containing 0.2% HCOOH) as described for the HPLC analysis of root samples. Retention times and (M+H)⁺ ions were as follows: morphine (7.8 min; *m*/*z* 286.1); dihydrocodeine (10.7 min; *m*/*z* 302.1); codeine (11.0 min; *m*/*z* 300.1); oripavine (12.4 min; *m*/*z* 298.1); reticuline (14.1 min; *m*/*z* 330.1); scoulerine, 1,2-dehydroreticuline, salutaridine (14.8 min; *m*/*z* 328.1); laudanine (15.7 min; *m*/*z* 344.1); thebaine (17.0 min; *m*/*z* 312.1) laudanosine (17.5 min; *m*/*z* 358.1).

The MS-spectra of the alkaloids salutaridine, 1,2-dehydroreticuline and scoulerine were characterized by the molecular ion peak at m/z 328.1. To distinguish between these three compounds the selected reaction monitoring (SRM) mode was applied. The positive ion electrospray (ES) selected reaction monitoring data were obtained from a Finnigan MAT TSQ 7000 instrument (electrospray voltage 4.5 V; APICID offset voltage 10V, heated capillary temperature 220°C; sheath gas: nitrogen) coupled with a Surveyor MicroLC system (Finnigan) equipped with a RP-18 column (4µm, 1 x 100 mm Ultrasep). For the HPLC a gradient system was used starting from H₂O : CH₃CN 85 : 15 (each of them containing 0.2% HOAc) to 5 : 95 within 30 min followed by a 10 min isocratic period; flow rate 70 µl min⁻¹. The following reactions based on the most prominent ion in the corresponding electrospray. CIDMS were used both for identification and quantification. A brief description of the SRM-method is given by Niessen (1999).

Full scan CIDMS data of the alkaloids (Raith *et al.,* 2003):
**dehydroreticuline:** positive ion electrospray CIDMS, *m*/*z* (rel. int.%): 328 ([M]⁺, 11), 313 (13), 312 ([M-CH₃-H]⁺, 100), 296 (6), 284 (41), 267 (7), 204 (5), 190 (10), 176 (8).
**salutaridine:** positive ion electrospray CIDMS, *m*/*z* (rel. int.%): 328 ([M+H]⁺, 37), 297 (11), 285 (23), 282 (26), 270 (34), 265 (35), 255 (22), 239 (41), 237 (100), 233 (18), 211 (45), 207 (39), 205 (18), 183 (15), 58 (50), 44 (14). **scoulerine:** positive ion electrospray CIDMS, *m*/*z* (rel. int.%): 328 ([M+H]⁺, 40), 296 (3), 178 (100), 176 (3), 151 (15).

### Example 2 : Results and Discussion

The presence of the transgene in regenerated *P. somniferum* plants was confirmed by the polymerase chain reaction (PCR) using genomic DNA as template and the appropriate oligodeoxynucleotide primers as given in the Material and Methods. Both the alkaloid biosynthetic gene *cyp80b1* encoding *(S)-N*-methylcoclaurine 3'-hydroxylase and the kanamycin resistance gene *nptll* encoding neomycin phosphotransferase type II were detected in genomic DNA isolated from T₃ generation transgenic plants (Figure 2). Introduction of the *sense-cyp80b*1 cDNA into *P. somniferum* by *Agrobacterium-mediated* transformation resulted in an overall increase of total alkaloids that was detectable in the T₁ generation and stable through at least the T₃ generation (Figure 3). In some cases, as shown in Figure 3, the relative ratios of the individual alkaloids remained constant, in others, such as shown in Figure 4, this ratio was altered. Specific to the transgenic plants shown in Figure 4, the relative amount of morphine was increased with respect to the other alkaloids, and the total alkaloid content was increased as well.

An important control in this type of metabolic engineering experiment is to demonstrate that the altered alkaloid profile is related to the presence of the transgene. To address this question, *P*. *somniferum* was transformed with the *antisense-cyp80b1* cDNA. This resulted in an overall decrease in the amount of alkaloid present in the T₁ generation and this phenotype was stable through at least the T₃ generation (Figure 5). In the example shown in Figure 5, the ratio of alkaloids was also altered.

In summary, the cytochrome P-450-dependent monooxygenase CYP80B1 that lies on the biosynthetic pathway to the benzylisoquinoline alkaloid branchpoint intermediate (S)-reticuline appears to limit the ability of *P. somniferum* to synthesize alkaloid. Overexpression of the cDNA *cyp80b1* in this plant resulted in an up to four-fold increase in the amount of total alkaloid in latex. This increase occurred either without changing the ratio of the individual alkaloids, or together with an overall increase in the ratio of morphine. Correspondingly, *antisense-cyp80b1* expressed in *P. somniferum* resulted in a reduction of total alkaloid in latex, suggesting that the observed phenotypes were dependent on the presence of the transgene. Transformation with *cyp80b1* can thus be used to modulate the alkaloid productivity of *P. somniferum* narcotic and condiment / oilseed cultivars.

### References

Birnboim HC and Dolly J (1979) A rapid alkaline extraction procedure for screening recombinant plasmid DNA. *Nucl. Acids Res.* **7**, 1513-1523
Brownstein MJ (1993) A brief history of opiates, opioid peptides and opioid receptors. *Proc. Natl. Acad. Sci.* **90**, 5391-5393
Cathala G, Savouret J-F, Mendez B, West BL, Karin M, Martial JA, and Baxter JD (1983) A method for isolation of intact translationally active ribonucleic acid. *DNA* **2** (4), 329-335
Chitty JA, Allen RS, Fist AJ and Larkin PJ (2003) Genetic transformation in commercial Tasmanian cultivars of opium poppy, *Papaver somniferum,* and movement of transgenic pollen in the field. *Functional Plant Biology* **30** (10), 1045-1058
Decker G, Wanner G, Zenk MH and Lottspeich F (2000) Characterization of the proteins in latex of the opium poppy *(Papaver somniferum)* using two-dimensional gel electrophoresis and microsequencing. *Electrophoresis* **21**, 3500-3516
Dieu P and Dunwell JM (1988) Anther culture with different genotypes of opium poppy *(Papaver somniferum* L.): effect of cold treatment. *Plant Cell, Tissue and Organ Culture* **12**, 263-271
Gamborg OL, Miller RA and Ojima K (1968) Nutrient requirements of suspension cultures of soybean root cells. *Exp. Cells Res.* **50**, 151-158
Huang FC and Kutchan TM (2000) Distribution of morphinan and benzo[c]phenanthridine alkaloid gene transcript accumulation in *Papaver somniferum. Phytochemistry* **53**, 555-564
Husain A and Sharma JR (eds.) (1983) The opium poppy. A Monograph. Central Institute of Medicinal and Aromatic Plants, Lucknow, India
Kassem A and Jacquin A (2001) Somatic embryogenesis, rhizogenesis and morphinan alkaloids production in two species of opium poppy (2001) *Journal of Biomedicine and Biotechnology* **1**, 70-78
Kutchan TM (1998) Molecular genetics of plant alkaloid biosynthesis. In: *The alkaloids* Vol. **50**, (ed. G. Cordell) Academic Press, San Diego, 253-316.
Larkin PJ, Chitty JA and Brettell RIS (1999) Methods for plant transformation and regeneration. International patent publication number WO 99/34663
Lazo GR, Stein PA and Ludwig RA (1991) A DNA transformation-competent *Arabidopsis* genomic library in *Agrobacterium. Bio*/*Technology* **9**, 963-967
Maniatis T, Fritsch EF, and Sambrook J (eds.) (1982) Molecular cloning: A laboratory manual. Cold Spring Harbor Laboratory Press, Cold Spring Harbor, New York
Nessler CL (1982) Somatic embryogenesis in the opium poppy *Papaver somniferum. Physiologia Plantarum* 55, 453-458
Niessen WMA (1999) Liquid Chromatography-Mass Spectrometry, Chapter 2: In *Introduction to Mass Spectrometry,* 67-69, second edition, Marcel Decker 1999
Ovecka M, Bobak M, Erdelsky K, Samaj J, Blehova A and Kristin J (1996) Morphology and conversion ability of somatic embryos in long term embryogenic callus culture of *Papaver somniferum. Biologia* **51**, 417-423
Pauli, HH and Kutchan, TM (1998) Molecular cloning and functional heterologous expression of two alleles encoding (*S*)-*N*-methylcoclaurine 3'-hydroxylase (CYP80B1), a new methyl jasmonate-inducible cytochrome P-450 dependent monooxygenase of benzylisoquinoline alkaloid biosynthesis. *Plant J.* **13**, 793-801
Park SU and Facchini P (2000) *Agrobacterium-mediated* transformation of opium poppy, *Papaver somniferum,* via shoot organogenesis. J. *Plant Physiol.* **157**, 207-214
Raith K, Neubert R, Poeaknapo C, Boettcher C, Zenk MH and Schmidt J (2003) Electrospray tandem mass spectrometric investigations of morphinans. J. *Am.* Soc. *Mass Spectrom. **14**,* 1262-1269
Schuchmann R and Wellmann E (1983) Somatic embryogenesis of tissue cultures of *Papaver somniferum* und *Papaver orientale* and its relationship to alkaloid and lipid matabolism. *Plant Cell Rep.* **2**, 88-91
Wakhlu AK and Bajwa PS (1986) Regeneration of uniform plants from somatic embryos of *Papaver somniferum* (opium poppy). *Phytomorphology* **361**, 101-105
Ye K, Ke Y, Keshava N, Shanks J, Kapp JA, Tekmal RR, Petros J and Joshi HC (1998) Opium alkaloid noscapine is an antitumor agent that arrests metaphase and induces apoptosis in dividing cells. *Proc. Natl. Acad.* Sci. **95**, 1601-1606
Yoshikawa T and Furuya T (1983) Regeneration and *in vitro* flowering of plants derived from callus cultures of opium poppy *(Papaver somniferum). Experientia* **39**: 1031-1033

## Claims

1. Method for altering alkaloid biosynthesis in a plant, said method comprising modifying the expression of an (*S*)-*N*-methylcoclaurine 3'-hydroxylase gene in the plant.

2. Method according to claim 1 wherein the modifying comprises induction, enhancement, suppression or inhibition of expression of endogenous *(S)-N*-methylcoclaurine 3'-hydroxylase gene sequences in the plant.

3. Method for altering alkaloid biosynthesis in a plant, comprising :
i) introducing into cells of a plant, an expressible exogenous nucleic acid comprising or consisting of an *(S)-N-*methylcoclaurine 3'-hydroxylase gene, or a derivative thereof, and
ii) optionally propagating the plant,
wherein expression of the exogenous nucleic acid in the plant or in its progeny results in altered levels of alkaloid biosynthesis.

4. Method according to claim 3 wherein expression of the exogenous nucleic acid gives rise to over-expression of (*S*)-*N*-methylcoclaurine 3'-hydroxylase in the plant.

5. Method according to claim 3 wherein expression of the exogenous nucleic acid gives rise to decreased or abolished expression of (*S*)-*N*-methylcoclaurine 3'-hydroxylase in the plant.

6. Method according to any one of claims 1 to 5 wherein the plant is a plant producing endogenous benzylisoquinoline-derived alkaloids.

7. Method according to claim 6 wherein the plant is a plant belonging to the order Ranunculales.

8. Method according to claim 7 wherein the plant is a plant belonging to the family Papaveraceae, Euphorbiaceae, Berberidaceae, Fumariaceae or Ranunculaceae.

9. Method according to claim 8 wherein the plant belongs to the genus Papaver.

10. Method according to claim 9 wherein the plant belongs to a species selected from *Papaver somniferum, Papaver bracteatum, Papaver setigerum, Papaver orientale, Papaver pseudo-orientale, Papaver cylindricum,* or *Papaver rhoeas.*

11. Method according to any one of claims 3 to 10, wherein the exogenous nucleic acid is a *cyp80b1* gene of *Papaver somniferum* or a *cyp80b1* gene of *Eschscholzia californica.*

12. Method according to claim 11 wherein the *cyp80b1* gene encodes a *(S)*-*N*-methylcoclaurine 3'-hydroxylase protein having the sequence illustrated in Figure 6 (SEQ. ID n°1).

13. Method according to claim 3 wherein the exogenous nucleic acid is a derivative of an (*S*)-*N-*methylcoclaurine 3'-hydroxylase gene, said derivative comprising a single or double stranded sequence variant, a fragment, a complementary sequence, an RNA equivalent, a mixed DNA/RNA equivalent, or an analogue of said gene.

14. Method according to claim 13, wherein the derivative is a derivative of a *cyp80b1* gene of *Papaver somniferum.*

15. Method according to claim 14, wherein the derivative is chosen from :
i) a sequence encoding the protein sequence illustrated in Figure 6,
ii) a variant of the nucleotide sequence illustrated in Figure 6, said variant having at least 70% identity with the sequence of Figure 6 over a length of at least 1000 bases, and encoding a *(S)-N*-methylcoclaurine 3'-hydroxylase, or
iii) a fragment of sequence (i) or (ii), said fragment having a length of at least 20 nucleotides, or
iv) a sequence complementary to any one of sequences (i), (ii) or (iii), and having a length of at least 20 nucleotides, or
v) any one of sequences (i), (ii), (iii) or (iv) in double-stranded form, or
vi) the RNA equivalent of any of sequences (i), (ii), (iii), (iv) or (v).

16. Method according to claim 15, wherein the derivative is a variant of the coding sequence illustrated in Figure 6, wherein said variant has at least 85% identity with the sequence of Figure 6 over a length of at least 1000 bases, and differs from the sequence of Figure 6 by insertion, replacement and / or deletion of at least one nucleotide.

17. Method according to claim 16, wherein the derivative is a variant comprising a coding sequence as illustrated in Figure 11 or Figure 13 or a portion of said coding sequence.

18. Method according to claim 16, wherein the variant has the capacity to hybridise to the sequence illustrated in Figure 6 in stringent conditions.

19. Method according to claim 18, wherein the variant has at least 99% identity with the nucleotide sequence of Figure 6 over a length of at least 1000 bases, and differs from the sequence of Figure 6 by insertion, replacement and / or deletion of at least one nucleotide.

20. Method according to claim 19, wherein the variant comprises an allelic variant of the *cyp80b1* gene of *Papaver somniferum* illustrated in Figure 7, or a portion thereof.

21. Method according to claim 15, wherein the variant is a hybrid sequence comprising a portion of the *cyp80b1* gene of *P. somniferum* and a portion of a *cyp80b1* gene of a plant species other than *P.somniferum.*

22. Method according to claim 15, wherein the derivative consists of, or comprises a fragment of the nucleotide sequence illustrated in Figure 6, said fragment having a length of 25 to 1400 nucleotides.

23. Method according to claim 22, wherein the derivative consists of, or comprises a fragment of the nucleotide sequence illustrated in Figure 6, said fragment having a length of 60 to 500 nucleotides.

24. Method according to claim 15, wherein the derivative consists of, or comprises a sequence which is complementary to the nucleotide sequence illustrated in Figure 6 or to a part thereof, or to the sequence illustrated in Figure 11 or a part thereof, or to the sequence illustrated in Figure 13 or a part thereof.

25. Method according to claim 15, wherein the derivative comprises an antisense sequence, a ribozyme sequence, a DNAzyme sequence, an RNA-interference sequence, or a sequence capable of giving rise to an antisense sequence, a ribozyme sequence, a DNAzyme sequence, an RNA-interference sequence or the complement of an antisense sequence, a ribozyme sequence, a DNAzyme sequence, an RNA-interference sequence.

26. Method according to any one of claims 15 to 25 wherein the derivative comprises at least one nucleotide analogue.

27. Method according to any one of claims 1 to 26, wherein the derivative comprises a chimeric gene containing heterologous regulatory signals.

28. Method according to any one of claims 1 to 5 wherein the altered alkaloid biosynthesis affects levels of alkaloids of the morphine and laudanine biosynthetic pathways.

29. Method according to claim 28, wherein the altered alkaloid biosynthesis affects levels of at least one alkaloid selected from the group consisting of morphine, codeine, codeinone, thebaine, oripavine, reticuline, (S)-laudanine and laudanosine.

30. Method according to claim 28 or 29 wherein the plant is a plant of the species Papaver, and the altered alkaloid biosynthesis results in an increase in the amount of total alkaloid in latex of the plant.

31. Method according to claim 28 or 29 wherein the plant is a plant of the species Papaver, and the altered alkaloid biosynthesis results in a decrease in the amount of total alkaloid in latex of the plant.

32. Method according to claim 28 or 29 wherein the plant is a plant of the species Papaver and the altered alkaloid biosynthesis results in a change in the proportions of the individual alkaloids in the latex of the plant.

33. Method according to claim 32 wherein the altered alkaloid biosynthesis results in an increase in the proportion of the morphine levels in the latex of the plant.

34. Method according to claim 32 wherein the amount of total alkaloid in latex of the plant remains unchanged.

35. Method according to any one of claims 3 to 34 wherein the plant is *P.somniferum,* and the exogenous nucleic acid comprises or consists of a sense sequence encoding the *P.somniferum* CYP80B1 protein illustrated in Figure 6.

36. Method according to any one of claims 3 to 34 wherein the plant is *P.somniferum,* and the exogenous nucleic acid comprises or consists of an antisense sequence of a sequence encoding the *P.somniferum* CYP80B1 protein illustrated in Figure 6.

37. Method according to any one of claims 3 to 36 wherein the exogenous nucleic acid is introduced into the plant genome by means of transformation with Agrobacterium.

38. Method according to claim 37 wherein the Agrobacterium is *Agrobacterium tumefaciens.*

39. Method for producing a plant having altered alkaloid biosynthesis, comprising
i) modifying the expression of an *(S)*-*N*-methylcoclaurine 3'-hydroxylase gene in the plant by a method according to any one of claims 1 to 38, and
ii) optionally propagating the plant.

40. Method according to claim 39 wherein the propagation of the plant is effected by means of self-fertilisation or cross-fertilisation, followed by selection of progeny plants having the capacity to exhibit altered alkaloid biosynthesis.

41. Method according to claim 39 wherein the propagation of the plant is effected by means of clonal propagation.

42. Method according to any one of claims 39 to 41 wherein the plant is *P.somniferum.*

43. Method for producing alkaloids comprising :
i) producing a plant having altered alkaloid biosynthesis, by the method of any one of claims 39 to 42,
ii) harvesting the plant parts containing the alkaloids,
iii) extracting the alkaloids from the plant parts.

44. Method according to claim 43, wherein the plant is *P.somniferum* and the plant parts which are harvested are poppy capsules or straw or latex, and the alkaloids extracted are selected from the group consisting of morphine, codeine, codeinone, thebaine, oripavine, reticuline, (S)-laudanine and laudanosine.

45. Plant having altered alkaloid biosynthesis, said plant containing in its genetic material, an exogenous nucleic acid comprising or consisting of an (*S*)-*N*-methylcoclaurine 3'-hydroxylase gene, or a derivative thereof.

46. Plant according to claim 45 wherein the exogenous nucleic acid is heterologous with respect to the genome of the plant.

47. Plant according to claim 46 wherein the exogenous nucleic acid comprises heterologous transcription regulatory signals.

48. Plant according to claim 47 wherein the regulatory signals permit expression of the exogenous nucleic acid in the vascular bundle of the plant.

49. Plant according to any one of claims 45 to 48 which is *P.somniferum.*

50. Plant according to any one of claims 45 to 49 wherein the exogenous nucleic acid is stably integrated into the genome of the plant.

51. Plant cell containing in its genetic material, an exogenous nucleic acid comprising or consisting of an *(S)-N-*methylcoclaurine 3'-hydroxylase gene, or a derivative thereof.

52. Nucleic acid encoding a (*S)-N-*methylcoclaurine 3'-hydroxylase, said nucleic acid comprising the *P.somniferum cyp80b1* coding sequence illustrated in Figure 7.

53. Nucleic acid encoding a *(S)-N*-methylcoclaurine 3'-hydroxylase comprising a portion of the *P.somniferum cyp80b1* coding sequence illustrated in Figure 7 and a portion of the *E.californica cyp80b1* coding sequence illustrated in Figure 11 or Figure 13.

54. Nucleic acid comprising or consisting of the 5' regulatory sequences of the *P.somniferum cyp80b1* gene illustrated in Figure 9.

55. Chimeric gene comprising the 5' regulatory sequences of the *P.somniferum cyp80b1* gene illustrated in Figure 9.


SEQUENCE LISTING

<110> LEIBNIZ INSTITUT FÜR PFLANZENBIOCHEMIE
LEIBNIZ INSTITUT FÜR PFLANZENBIOCHEMIE IPB

<120> Modulation of alkaloid biosynthesis in plants and plants having altered
alkaloid biosynthesis.

<130> B6130

<140> EP 04019154.6
<141> 2004-08-12

<160> 9

<170> PatentIn version 3.1

<210> 1
<211> 1528
<212> DNA
<213> Papaver somniferum

<221> CDS
<222> (1)..(1464)

<400> 1
atg gag atc gtc aca gta tca ctt gta gca gtt gtg atc act act ttc 48
Met Glu Ile Val Thr Val Ser Leu Val Ala Val Val Ile Thr Thr Phe
1               5                   10                  15

tta tac tta atc ttc aga gat tca agt cct aaa ggt ttg cca cca ggt 96
Leu Tyr Leu Ile Phe Arg Asp Ser Ser Pro Lys Gly Leu Pro Pro Gly
            20                  25                  30

cca aaa ccc tgg cca ata gtt gga aac ctt ctt caa ctt ggt gag aaa 144
Pro Lys Pro Trp Pro Ile Val Gly Asn Leu Leu Gln Leu Gly Glu Lys
        35                  40                  45

cct cat tct cag ttt gct cag ctt gct gaa acc tat ggt gat ctc ttt 192
Pro His Ser Gln Phe Ala Gln Leu Ala Glu Thr Tyr Gly Asp Leu Phe
    50                  55                  60

tca ctg aaa cta gga agt gaa acg gtt gtt gta gct tca act cca tta 240
Ser Leu Lys Leu Gly Ser Glu Thr Val Val Val Ala Ser Thr Pro Leu
65                  70                  75                  80

gca gct agc gag att cta aag acg cat gat cgt gtt ctc tct ggt cga 288
Ala Ala Ser Glu Ile Leu Lys Thr His Asp Arg Val Leu Ser Gly Arg
                85                  90                  95

tac gtg ttt caa agt ttc cgg gta aaa gaa cat gtg gag aac tct att 336
Tyr Val Phe Gln Ser Phe Arg Val Lys Glu His Val Glu Asn Ser Ile
            100                 105                 110

gtg tgg tct gaa tgt aat gaa aca tgg aag aaa ctg cgg aaa gtt tgt 384
Val Trp Ser Glu Cys Asn Glu Thr Trp Lys Lys Leu Arg Lys Val Cys
        115                 120                 125

aga acg gac ctt ttt acg cag aag atg att gaa agt caa gct gaa gtt 432
Arg Thr Asp Leu Phe Thr Gln Lys Met Ile Glu Ser Gln Ala Glu Val
    130                 135                 140

aga gaa agt aag gct atg gaa atg gtg gag tat ttg aag aaa aat gta     480
Arg Glu Ser Lys Ala Met Glu Met Val Glu Tyr Leu Lys Lys Asn Val
145                 150                 155                 160

gga aat gaa gtg aaa att gct gaa gtt gta ttt ggg acg ttg gtg aat     528
Gly Asn Glu Val Lys Ile Ala Glu Val Val Phe Gly Thr Leu Val Asn
                165                 170                 175

ata ttc ggt aac ttg ata ttt tca caa aat att ttc aag ttg ggt gat     576
Ile Phe Gly Asn Leu Ile Phe Ser Gln Asn Ile Phe Lys Leu Gly Asp
            180                 185                 190

gaa agt agt gga agt gta gaa atg aaa gaa cat cta tgg aga atg ctg     624
Glu Ser Ser Gly Ser Val Glu Met Lys Glu His Leu Trp Arg Met Leu
        195                 200                 205

gaa ttg ggg aac tcg aca aat cca gct gat tat ttt cca ttt ttg ggt     672
Glu Leu Gly Asn Ser Thr Asn Pro Ala Asp Tyr Phe Pro Phe Leu Gly
    210                 215                 220

aaa ttc gat ttg ttt gga caa agc aaa gat gtt gct gat tgt ctg caa     720
Lys Phe Asp Leu Phe Gly Gln Ser Lys Asp Val Ala Asp Cys Leu Gln
225                 230                 235                 240

ggg att tat agt gtt tgg ggt gct atg ctc aaa gaa agc aaa ata gcc     768
Gly Ile Tyr Ser Val Trp Gly Ala Met Leu Lys Glu Ser Lys Ile Ala
                245                 250                 255

aag cag cat aac aac agc aag aag aat gat ttt gtt gag att ttg ctc     816
Lys Gln His Asn Asn Ser Lys Lys Asn Asp Phe Val Glu Ile Leu Leu
            260                 265                 270

gat tcc gga ctc gat gac cag cag att aat gcc ttg ctc atg gaa ata     864
Asp Ser Gly Leu Asp Asp Gln Gln Ile Asn Ala Leu Leu Met Glu Ile
        275                 280                 285

ttt ggt gcg gga aca gag aca agt gca tct aca ata gaa tgg gcg ttg     912
Phe Gly Ala Gly Thr Glu Thr Ser Ala Ser Thr Ile Glu Trp Ala Leu
    290                 295                 300

tct gag ctc aca aaa aac cct caa gta aca gcc aat atg cgg ttg gaa     960
Ser Glu Leu Thr Lys Asn Pro Gln Val Thr Ala Asn Met Arg Leu Glu
305                 310                 315                 320

ttg tta tct gtg gta ggg aag agg ccg gtt aag gaa tcc gac ata cca    1008
Leu Leu Ser Val Val Gly Lys Arg Pro Val Lys Glu Ser Asp Ile Pro
                325                 330                 335

aac atg cct tat ctt caa gct ttt gtt aaa gaa act cta cgg ctt cat    1056
Asn Met Pro Tyr Leu Gln Ala Phe Val Lys Glu Thr Leu Arg Leu His
            340                 345                 350

cca gca act cct ctg ctg ctt cca cgt cga gca ctt gag acc tgc aaa    1104
Pro Ala Thr Pro Leu Leu Leu Pro Arg Arg Ala Leu Glu Thr Cys Lys
        355                 360                 365

gtt ttg aac tat acg atc ccg aaa gag tgt cag att atg gtg aac gcc    1152
Val Leu Asn Tyr Thr Ile Pro Lys Glu Cys Gln Ile Met Val Asn Ala
    370                 375                 380

tgg ggc att ggt cgg gat cca aaa agg tgg act gat cca ttg aag ttt     1200
Trp Gly Ile Gly Arg Asp Pro Lys Arg Trp Thr Asp Pro Leu Lys Phe
385                 390                 395                 400

tca cca gag agg ttc ttg aat tcg agc att gat ttc aaa ggg aac gac     1248
Ser Pro Glu Arg Phe Leu Asn Ser Ser Ile Asp Phe Lys Gly Asn Asp
                405                 410                 415

ttc gag ttg ata cca ttt ggt gca ggg aga agg ata tgt cct ggt gtg     1296
Phe Glu Leu Ile Pro Phe Gly Ala Gly Arg Arg Ile Cys Pro Gly Val
            420                 425                 430

ccc ttg gca act caa ttt att agt ctt att gtg tct agt ttg gta cag     1344
Pro Leu Ala Thr Gln Phe Ile Ser Leu Ile Val Ser Ser Leu Val Gln
        435                 440                 445

aat ttt gat tgg gga tta ccg aag gga atg gat cct agc caa ctg atc     1392
Asn Phe Asp Trp Gly Leu Pro Lys Gly Met Asp Pro Ser Gln Leu Ile
    450                 455                 460

atg gaa gag aaa ttt ggg ttg aca ctg caa aag gaa cca cct ctg tat     1440
Met Glu Glu Lys Phe Gly Leu Thr Leu Gln Lys Glu Pro Pro Leu Tyr
465                 470                 475                 480

att gtt cct aaa act cgg gat taa tctcaatcaa gaaatcttaa attcacatga     1494
Ile Val Pro Lys Thr Arg Asp
                485

ttatggattt ctcgctactt tatttgaaaa aaaa                                1528


<210>  2
<211>  487
<212>  PRT
<213>  Papaver somniferum

<400>  2

Met Glu Ile Val Thr Val Ser Leu Val Ala Val Val Ile Thr Thr Phe
1               5                   10                  15

Leu Tyr Leu Ile Phe Arg Asp Ser Ser Pro Lys Gly Leu Pro Pro Gly
            20                  25                  30

Pro Lys Pro Trp Pro Ile Val Gly Asn Leu Leu Gln Leu Gly Glu Lys
        35                  40                  45

Pro His Ser Gln Phe Ala Gln Leu Ala Glu Thr Tyr Gly Asp Leu Phe
    50                  55                  60

Ser Leu Lys Leu Gly Ser Glu Thr Val Val Val Ala Ser Thr Pro Leu
65                  70                  75                  80

Ala Ala Ser Glu Ile Leu Lys Thr His Asp Arg Val Leu Ser Gly Arg
                85                  90                  95

Tyr Val Phe Gln Ser Phe Arg Val Lys Glu His Val Glu Asn Ser Ile
            100                 105                 110

Val Trp Ser Glu Cys Asn Glu Thr Trp Lys Lys Leu Arg Lys Val Cys
        115                 120                 125

Arg Thr Asp Leu Phe Thr Gln Lys Met Ile Glu Ser Gln Ala Glu Val
    130                 135                 140

Arg Glu Ser Lys Ala Met Glu Met Val Glu Tyr Leu Lys Lys Asn Val
145                 150                 155                 160

Gly Asn Glu Val Lys Ile Ala Glu Val Val Phe Gly Thr Leu Val Asn
                165                 170                 175

Ile Phe Gly Asn Leu Ile Phe Ser Gln Asn Ile Phe Lys Leu Gly Asp
            180                 185                 190

Glu Ser Ser Gly Ser Val Glu Met Lys Glu His Leu Trp Arg Met Leu
        195                 200                 205

Glu Leu Gly Asn Ser Thr Asn Pro Ala Asp Tyr Phe Pro Phe Leu Gly
    210                 215                 220

Lys Phe Asp Leu Phe Gly Gln Ser Lys Asp Val Ala Asp Cys Leu Gln
225                 230                 235                 240

Gly Ile Tyr Ser Val Trp Gly Ala Met Leu Lys Glu Ser Lys Ile Ala
                245                 250                 255

Lys Gln His Asn Asn Ser Lys Lys Asn Asp Phe Val Glu Ile Leu Leu
            260                 265                 270

Asp Ser Gly Leu Asp Asp Gln Gln Ile Asn Ala Leu Leu Met Glu Ile
        275                 280                 285

Phe Gly Ala Gly Thr Glu Thr Ser Ala Ser Thr Ile Glu Trp Ala Leu
    290                 295                 300

Ser Glu Leu Thr Lys Asn Pro Gln Val Thr Ala Asn Met Arg Leu Glu
305                 310                 315                 320

Leu Leu Ser Val Val Gly Lys Arg Pro Val Lys Glu Ser Asp Ile Pro
                325                 330                 335

Asn Met Pro Tyr Leu Gln Ala Phe Val Lys Glu Thr Leu Arg Leu His
            340                 345                 350

Pro Ala Thr Pro Leu Leu Leu Pro Arg Arg Ala Leu Glu Thr Cys Lys
        355                 360                 365

Val Leu Asn Tyr Thr Ile Pro Lys Glu Cys Gln Ile Met Val Asn Ala
    370                 375                 380

Trp Gly Ile Gly Arg Asp Pro Lys Arg Trp Thr Asp Pro Leu Lys Phe
385                 390                 395                 400

Ser Pro Glu Arg Phe Leu Asn Ser Ser Ile Asp Phe Lys Gly Asn Asp
                405                 410                 415

Phe Glu Leu Ile Pro Phe Gly Ala Gly Arg Arg Ile Cys Pro Gly Val
            420                 425                 430

Pro Leu Ala Thr Gln Phe Ile Ser Leu Ile Val Ser Ser Leu Val Gln
        435                 440                 445

Asn Phe Asp Trp Gly Leu Pro Lys Gly Met Asp Pro Ser Gln Leu Ile
    450                 455                 460

Met Glu Glu Lys Phe Gly Leu Thr Leu Gln Lys Glu Pro Pro Leu Tyr
465                 470                 475                 480

Ile Val Pro Lys Thr Arg Asp
                485

<210> 3
<211> 481
<212> PRT
<213> Papaver somniferum

<400> 3

Ser Leu Val Ala Val Val Ile Thr Thr Phe Leu Tyr Leu Ile Phe Arg
1               5                   10                  15

Asp Ser Ser Pro Lys Gly Leu Pro Pro Gly Pro Lys Pro Trp Pro Ile
            20                  25                  30

Val Gly Asn Leu Leu Gln Leu Gly Glu Lys Pro His Ser Gln Phe Ala
        35                  40                  45

Gln Leu Ala Glu Thr Tyr Gly Asp Leu Phe Ser Leu Lys Leu Gly Ser
    50                  55                  60

Glu Thr Val Val Val Ala Ser Thr Pro Leu Ala Ala Ser Glu Ile Leu
65                  70                  75                  80

Lys Thr His Asp Arg Val Leu Ser Gly Arg Tyr Val Phe Gln Ser Phe
                85                  90                  95

Arg Val Lys Glu His Val Glu Asn Ser Ile Val Trp Ser Glu Cys Asn
            100                 105                 110

Glu Thr Trp Lys Lys Leu Arg Lys Val Cys Arg Thr Asp Leu Phe Thr
        115                 120                 125

Gln Lys Met Ile Glu Ser Gln Ala Glu Val Arg Glu Ser Lys Ala Met
    130                 135                 140

Glu Met Val Glu Tyr Leu Lys Lys Asn Val Gly Asn Glu Val Lys Ile
145                 150                 155                 160

Ala Glu Val Val Phe Gly Thr Leu Val Asn Ile Phe Gly Asn Leu Ile
                165                 170                 175

Phe Ser Gln Asn Ile Phe Lys Leu Gly Asp Glu Ser Ser Gly Ser Val
            180                 185                 190

Glu Met Lys Glu His Leu Trp Arg Met Leu Glu Leu Gly Asn Ser Thr
        195                 200                 205

Asn Pro Ala Asp Tyr Phe Pro Phe Leu Gly Lys Phe Asp Leu Phe Gly
    210                 215                 220

Gln Ser Lys Asp Val Ala Asp Cys Leu Gln Gly Ile Tyr Ser Val Trp
225                 230                 235                 240

Gly Ala Met Leu Lys Glu Ser Lys Ile Ala Lys Gln His Asn Asn Ser
                245                 250                 255

Lys Lys Asn Asp Phe Val Glu Ile Leu Leu Asp Ser Gly Leu Asp Asp
            260                 265                 270

Gln Gln Ile Asn Ala Leu Leu Met Glu Ile Phe Gly Ala Gly Thr Glu
        275                 280                 285

Thr Ser Ala Ser Thr Ile Glu Trp Ala Leu Ser Glu Leu Thr Lys Asn
    290                 295                 300

Pro Gln Val Thr Ala Asn Met Arg Leu Glu Leu Leu Ser Val Val Gly
305                 310                 315                 320

Lys Arg Pro Val Lys Glu Ser Asp Ile Pro Asn Met Pro Tyr Leu Gln
                325                 330                 335

Ala Phe Val Lys Glu Thr Leu Arg Leu His Pro Ala Thr Pro Leu Leu
            340                 345                 350

Leu Pro Arg Arg Ala Leu Glu Thr Cys Lys Val Leu Asn Tyr Thr Ile
        355                 360                 365

Pro Lys Glu Cys Gln Ile Met Val Asn Ala Trp Gly Ile Gly Arg Asp
    370                 375                 380

Pro Lys Arg Trp Thr Asp Pro Leu Lys Phe Ser Pro Glu Arg Phe Leu
385                 390                 395                 400

Asn Ser Ser Ile Asp Phe Lys Gly Asn Asp Phe Glu Leu Ile Pro Phe
                405                 410                 415

Gly Ala Gly Arg Arg Ile Cys Pro Gly Val Pro Leu Ala Thr Gln Phe
            420                 425                 430

Ile Ser Leu Ile Val Ser Ser Leu Val Gln Asn Phe Asp Trp Gly Leu
        435                 440                 445

Pro Lys Gly Met Asp Pro Ser Gln Leu Ile Met Glu Glu Lys Phe Gly
    450                 455                 460

Leu Thr Leu Gln Lys Glu Pro Pro Leu Tyr Ile Val Pro Lys Thr Arg
465                 470                 475                 480

Asp

<210>  4
<211>  1511
<212>  DNA
<213>  Papaver somniferum

<400>  4
atcacttgta gcagttgtga tcactacttt cttatactta atcttcagag attcaagtcc      60

taaaggtttg ccaccaggtc caaaaccctg gccaatagtt ggaaaccttc ttcaacttgg     120

tgagaaacct cattctcagt ttgctcagct tgctgaaacc tatggtgatc tcttttcact 180

gaaactagga agtgaaacgg ttgttgtagc ttcaactcca ttagcagcta gcgagattct 240

aaagacgcat gatcgtgttc tctctggtcg atacgtgttt caaagtttcc gggtaaaaga 300

acatgtggag aactctattg tgtggtctga atgtaatgaa acatggaaga aactgcggaa 360

agtttgtaga acggaccttt ttacgcagaa gatgattgaa agtcaagctg aagttagaga 420

aagtaaggct atggaaatgg tggagtattt gaagaaaaat gtaggaaatg aagtgaaaat 480

tgctgaagtt gtatttggga cgttggtgaa tatattcggt aacttgatat tttcacaaaa 540

tattttcaag ttgggtgatg aaagtagtgg aagtgtagaa atgaaagaac atctatggag 600

aatgctggaa ttggggaact cgacaaatcc agctgattat tttccatttt tgggtaaatt 660

cgatttgttt ggacaaagca aagatgttgc tgattgtctg caagggattt atagtgtttg 720

gggtgctatg ctcaaagaaa gcaaaatagc caagcagcat aacaacagca agaagaatga 780

ttttgttgag attttgctcg attccggact cgatgaccag cagattaatg ccttgctcat 840

ggaaatattt ggtgcgggaa cagagacaag tgcatctaca atagaatggg cgttgtctga 900

gctcacaaaa aaccctcaag taacagccaa tatgcggttg gaattgttat ctgtggtagg 960

gaagaggccg gttaaggaat ccgacatacc aaacatgcct tatcttcaag cttttgttaa 1020

agaaactcta cggcttcatc cagcaactcc tctgctgctt ccacgtcgag cacttgagac 1080

ctgcaaagtt ttgaactata cgatcccgaa agagtgtcag attatggtga acgcctgggg 1140

cattggtcgg gatccaaaaa ggtggactga tccattgaag ttttcaccag agaggttctt 1200

gaattcgagc attgatttca aagggaacga cttcgagttg ataccatttg gtgcagggag 1260

aaggatatgt cctggtgtgc ccttggcaac tcaatttatt agtcttattg tgtctagttt 1320

ggtacagaat tttgattggg gattaccgaa gggaatggat cctagccaac tgatcatgga 1380

agagaaattt gggttgacac tgcaaaagga accacctctg tatattgttc ctaaaactcg 1440

ggattaatct caatcaagaa atcttaaatt cacatgatta tggatttctc gctactttat 1500

ttgaaaaaaa a 1511

<210> 5
<211> 3816
<212> DNA
<213> Papaver somniferum

<400> 5
aaatttgtac ctattacgtc tcttctttct ggtataccag gcagtggcta tcttagtcat 60

gcgattaact tgtacctccg ctgtaagttc tttactgtgg atttgaaacg aattaaaact 120

actttatgtc tatccgttcc ttccatgctt aaattatatt ctcctcggac tcaattgagc 180

tgctcatcaa catgtttatt atttgacaat gcaaaccgat tcaaactagt attctccact 240
accacttgta gcatgactca ttttctggat gtatcgatct ctgaatgttc atttatattc 300
atgagaagtg cattgtgtct tcttttctct tttggctatt aatattctaa atcatatttg 360
gccgttagat aaacctgatc caaaggattt gcaatacttc ctggagttcc aagttccaag 420
gcagtgggca cctatgttta atgagctgcc tcttgggcac caaaggaggg cctcttgtcc 480
ttctttgcag ttcagtttca tgggatcaaa gcttcatgtc aactctacgc aggtacactc 540
tatttattct tggatatatc ttatttttac caaaatactc ttggttgtgc tgtactactg 600
tctaaaacaa tcctggttgt ttagttgtgt tttacttaag cgaggtctta tgaagagttc 660
caaacagagt ttaggctcga atagaagatc caaaaacaat agattagaaa aggtgaacta 720
ggatatagta gtttttgggt atatcatttc gttttttcat agaacactga gagattggta 780
gaagactgtc tcaacgcgac cttaacatgc ttgtataacc tggctcagag cttgctttat 840
attaacctgc ttgttgagtt tcaaacttta gtcctgtgtg ctttcacggc acctgaaacc 900
tataataatt gttctgaaac aaaataatgt taatacttgt acaaatactg aggctaacat 960
taagtaaatt tgttatgttt tgagcatatt atttaacaag gggttagcag agatagattg 1020
gagaaaatag gcttgatcat atgggtaaga gttaggttga ttcatcttaa cctgtctaca 1080
ttatctccta gccaatgcat ataagaaaaa aggatcaaat cgtaattgat ttactccata 1140
tttcaaataa tggttaaacc gacagaacat aaggttttat aaaggtggtt tgcccttcta 1200
aatcctgctc ccatagataa agctttaagt acaaataccg ttttgttaat aaaatatagt 1260
aataatttgt ttttttgcta aactgatgct tttagcatgc ttcagaaggc aggctattct 1320
aaaatgattt tagcatgctt cttaaagcag gctgttaata tttgaagctt tcttaagcat 1380
tgatgccatc ataataaaag agggaggtcc cattgacatt gttaaattta tactatcgac 1440
attttttatg ctgaagtttt tttaatattc aagtgaaaat agtcggaagc ttactgttca 1500
tatttaacaa tcttcttgat gtggtacact ctcgcaaaaa attagcgcat ttcatgactt 1560
gtaccctaac tttttaccag tttgaaactc aactgtttgt tagcaacaat tgaaatcaaa 1620
accagtaaat gatggataca tactacgaaa tgcactaatt tttggtggga gtggagtgca 1680
taatgagaag agcatcatac ctaaagaatt agattataac tagtttttat ttgaaccgca 1740
taaaaaaact agggtcgaat gtgtaagata gtgtcaattt aacaatgtca acggaacctc 1800
tttcatatgt aaagtatcat cacatgataa gcaaaacata tatatgactt ccggagcctc 1860
agtaggatca atgtagttat taccaatttc aattacatta cctggtagga tcacaatcat 1920
taatatttta ggttaagagc agaatttgtc gtgtatgact tgtattaata aatttttggc 1980

gtcaattcta ggtctcaagt ggcaggactc ctgtggtagg tcttcgtctt tacctagaag 2040

ggaaggcatg caaccgcttg gccatacatg tacagcacct ctcgaacctt ccaaggttac 2100

ttgaatcttc atgggctgac cccactacgc tgaaacaatg tcaatggcga gggtctgatg 2160

aatccagtgt cccatactta gaacccataa aatggaaaag atactccaac atttgcacat 2220

cagtcgtaaa acatgatcca ggttggttgc atggagaatc accaggtgta ttcattgtca 2280

ctggagctca actcatgacc aaggggaaat ggccaaaaca cattcttcac cttcgtttac 2340

tctacacaca tattcctggt ttcagcatcc agaaaacaga atgggcagtt gcaccagcat 2400

cttcacaaaa atcaagtttc ctcgccaatt tcagcacaac tttcacgact tcaggtcctt 2460

caaaggcagc tcctgtggta gctataaatt ccggagtgta ttctgatggc cctccaagac 2520

cagttcactc tcaaaagctt cttaaatatg ttgagacgtc agaaatagtc cgggggccac 2580

acgatattcc cggacactgg atggtaattg ctgctaagct agttacagaa ggtggcaaga 2640

ttggtttact tgtaaaattt gcattgcttg attattccag tcccacgagt gagtagaaga 2700

aagtttgatc cacattggat ctcacctaaa tctccccatc tatcctcctg aagagaagtt 2760

tgtttcaaac tccggttcga agaatacccc atgctaatgt aatgtaagta ttataatgga 2820

tccgtagtta ttgaagtttt tcgaccaact ctactttgtc gctggggata gttactataa 2880

agctttagaa gtaagaaacc gtgtacagaa atctcaaggc ctccccctct tcctttttct 2940

tttttttttt cttctctcat taaatttaat ctgtatgtaa attgagaagt tatagagttt 3000

ggttggtggt ttcaaataat gtattttcat ttaagcttat tatgattata tcatctctga 3060

gtatcaattc cacttgactc ttaactgtga aatcagaaca tgacatatgg gtgccttgaa 3120

aggttattca tatcctggga cttcttgtac atgtaagttg aaaacatcaa cttcattcaa 3180

agaaattatt agaaaaaacc ttccaacagt ctatctggat tagattccat gttgaatcaa 3240

ccactatttt gtacacctac aggcaggcta ctgttccata tgttagcatg tggatatgca 3300

caatttgaca agttttgtct gttaaaccaa aaattattca aaaaagttct cccacgcacc 3360

cccttaaaaa aatgcccaca tgcatctcgt gtgtagcact tgacaatcca cgagtgccat 3420

ccaaattgaa tttccctaca agattctgga catacttgct ctatgaaact taacttcttt 3480

atataaacaa ccaaagaaaa atacattgag agaaaatatg gagatcgtca cagtatcact 3540

tgtagcagtt gtgatcacta ctttcttata cttaatcttc agagattcaa gtcctaaagg 3600

tttgccacca ggtccaaaac cctggccaat agttggaaac cttcttcaac ttggtgagaa 3660

acctcattct cagtttgctc agcttgctga aacctatggt gatctctttt cactgaaact 3720

aggaagtgaa acggttgttg tagcttcaac tccattagca gctagcgaga ttctaaagac 3780

gcatgatcgt gttctctctg gtcgatacgt gtttca 3816

<210>  6
<211>  487
<212>  PRT
<213>  Eschscholzia californica

<400>  6

Gly Thr Ser Thr Val Ala Leu Ile Ala Val Ile Ile Ser Ser Ile Leu
1               5                   10                  15

Tyr Leu Leu Phe Gly Gly Ser Gly His Lys Asn Leu Pro Pro Gly Pro
            20                  25                  30

Lys Pro Trp Pro Ile Val Gly Asn Leu Leu Gln Leu Gly Glu Lys Pro
        35                  40                  45

His Ala Gln Phe Ala Glu Leu Ala Gln Thr Tyr Gly Asp Ile Phe Thr
    50                  55                  60

Leu Lys Met Gly Thr Glu Thr Val Val Val Ala Ser Thr Ser Ser Ala
65                  70                  75                  80

Ala Ser Glu Ile Leu Lys Thr His Asp Arg Ile Leu Ser Ala Arg Tyr
                85                  90                  95

Val Phe Gln Ser Phe Arg Val Lys Gly His Val Glu Asn Ser Ile Val
            100                 105                 110

Trp Ser Asp Cys Thr Glu Thr Trp Lys Asn Leu Arg Lys Val Cys Arg
        115                 120                 125

Thr Glu Leu Phe Thr Gln Lys Met Ile Glu Ser Gln Ala His Val Arg
    130                 135                 140

Glu Lys Lys Cys Glu Glu Met Val Glu Tyr Leu Met Lys Lys Gln Gly
145                 150                 155                 160

Glu Glu Val Lys Ile Val Glu Val Ile Phe Gly Thr Leu Val Asn Ile
                165                 170                 175

Phe Gly Asn Leu Ile Phe Ser Gln Asn Ile Phe Glu Leu Gly Asp Pro
            180                 185                 190

Asn Ser Gly Ser Ser Glu Phe Lys Glu Tyr Leu Trp Arg Met Leu Glu
        195                 200                 205

Leu Gly Asn Ser Thr Asn Pro Ala Asp Tyr Phe Pro Met Leu Gly Lys
    210                 215                 220

Phe Asp Leu Phe Gly Gln Arg Lys Glu Val Ala Glu Cys Leu Lys Gly
225                 230                 235                 240

Ile Tyr Ala Ile Trp Gly Ala Met Leu Gln Glu Arg Lys Leu Ala Lys
                245                 250                 255

Lys Val Asp Gly Tyr Lys Ser Lys Asn Asp Phe Val Asp Val Cys Leu
            260                 265                 270

Asp Ser Gly Leu Asn Asp Tyr Gln Ile Asn Ala Leu Leu Met Glu Leu
        275                 280                 285

Phe Gly Ala Gly Thr Glu Thr Ser Ala Ser Thr Ile Glu Trp Ala Met
    290                 295                 300

Thr Glu Leu Thr Lys Asn Pro Lys Ile Thr Ala Lys Ile Arg Ser Glu
305                 310                 315                 320

Ile Gln Thr Val Val Gly Glu Arg Ser Val Lys Glu Ser Asp Phe Pro
                325                 330                 335

Asn Leu Pro Tyr Leu Glu Ala Thr Val Lys Glu Thr Leu Arg Leu His
            340                 345                 350

Pro Pro Thr Pro Leu Leu Leu Pro Arg Arg Ala Leu Glu Thr Cys Thr
        355                 360                 365

Ile Leu Asn Tyr Thr Ile Pro Lys Asp Cys Gln Ile Met Val Asn Ala
    370                 375                 380

Trp Gly Ile Gly Arg Asp Pro Lys Thr Trp Thr Asp Pro Leu Thr Phe
385                 390                 395                 400

Ser Pro Glu Arg Phe Leu Asn Ser Ser Val Asp Phe Arg Gly Asn Asp
                405                 410                 415

Phe Ser Leu Ile Pro Phe Gly Ala Gly Arg Arg Ile Cys Pro Gly Leu
            420                 425                 430

Pro Ile Ala Asn Gln Phe Ile Ala Leu Leu Val Ala Thr Phe Val Gln
        435                 440                 445

Asn Leu Asp Trp Cys Leu Pro Asn Gly Met Ser Val Asp His Leu Ile
    450                 455                 460

Val Glu Glu Lys Phe Gly Leu Thr Leu Gln Lys Glu Pro Pro Leu Phe
465                 470                 475                 480

Ile Val Pro Lys Ser Arg Val
                485

<210>  7
<211>  1901
<212>  DNA
<213>  Eschscholzia californica

<400>  7
cggcacgagc acagtagcac ttattgcagt aataatttct tcaatactat acctcctctt      60

tggtggtagt ggtcacaaaa atctcccacc aggaccaaaa ccatggccaa tagttggaaa     120

tctcctccaa cttggtgaga aaccacacgc tcaattcgcc gaactagctc aaacctatgg     180

tgacattttc actcttaaaa tgggtactga aactgtagtt gttgcatcaa catcttcagc     240

agcttccgaa atactaaaaa cccatgatcg aattctatcc gctcgttacg tttttcaaag     300

ttttcgagta aaagggcatg tagaaaattc aatagtttgg tcagattgta ctgaaacttg     360

gaagaattta agaaaagttt gtaggacaga acttttcaca cagaagatga tagaaagtca     420

agctcatgtt agagagaaaa aatgtgaaga aatggttgaa tacttgatga aaaaacaagg     480

ggaagaagtg aaaattgtgg aagtaatatt tggaacatta gtgaatatat ttggaaattt     540

gatattttca cagaatatat ttgaattggg tgatccaaat agtggaagtt cagagttcaa     600

ggaatatcta tggaggatgt tggaattggg gaattcaaca aatccagctg attattttcc     660

aatgttaggt aaatttgatt tgtttggaca gaggaaagaa gttgcagagt gtttaaaagg     720

gatttatgca atatggggag ctatgcttca agaaagaaaa ttagctaaaa aagttgatgg     780

atataaaagc aagaatgatt ttgttgatgt ttgtcttgat tctggactta atgattatca     840

aatcaatgcc ttgcttatgg aattatttgg ggcaggcaca gaaacgagcg catcgacaat     900

tgagtgggcc atgactgaac taacaaagaa tccaaagata acagctaaga ttagatcaga     960

aattcaaaca gtggtaggcg agagatcggt aaaagaatcc gacttcccca atcttccata    1020

ccttgaagct actgttaaag aaaccctaag acttcaccca ccaactccat tgctactccc    1080

acgccgagca cttgaaacct gtacaatcct caactatacc atcccaaaag attgtcaaat    1140

tatggtcaac gcttggggaa tcggtcgtga tcccaagact tggaccgatc cgttgacttt    1200

ctcaccagag agattcttga attctagtgt tgactttagg gggaatgatt tcagtttgat    1260

accatttggt gcaggaagaa ggatatgccc cggtctgcca atagcaaatc agtttattgc 1320

attgctagtg gcaacatttg tgcaaaattt ggattggtgt ctaccaaatg ggatgagtgt 1380

tgaccatttg atagtggagg agaagtttgg gttgactctt caaaaagaac cacctctatt 1440

cattgttcct aaatcaaggg tttgatcttt ctcatcatgg ttcatgtgaa aactagatta 1500

tcttttgttt tggctatgca tctgttttct ttccttccta taagcttttg ttcaccaagt 1560

aagaaaatgt ccacacgatt tttgttcggc cgaggtccga gggtatcaag actatggaca 1620

acaatcctct gagacaacta aggggtcact ggatcaaaat tgtcgatatg tacattttct 1680

tctgaacaag gttacacatt ctagctatta ggtgtcgcgc taataaaata taataatcac 1740

ttttgatagt tttgtagaga catgttatgc aatacatatc tattgcaata cgcatgctca 1800

taatgcatat acatatttga aaagttgtgg atgttgttcc atgttcaacc ataaagcgag 1860

tgttttacta tgttaaagtg aaaaaaaaaa aaaaaaaaaa a 1901

<210> 8
<211> 488
<212> PRT
<213> Eschscholzia californica

<220>
<221> MISC_FEATURE
<222> (192)..(192)
<223> Xaa = UNKNOWN

<220>
<221> UNSURE
<222> (192)..(192)
<223> Xaa = UNKNOWN

<400> 8

Met Glu Val Val Thr Val Ala Leu Ile Ala Val Ile Ile Ser Ser Ile
1               5                   10                  15

Leu Tyr Leu Leu Phe Gly Ser Ser Gly His Lys Asn Leu Pro Pro Gly
            20                  25                  30

Pro Lys Pro Trp Pro Ile Val Gly Asn Leu Leu Gln Leu Gly Glu Lys
        35                  40                  45

Pro His Ala Gln Phe Ala Glu Leu Ala Gln Thr Tyr Gly Asp Ile Phe
    50                  55                  60

Thr Leu Lys Met Gly Thr Glu Thr Val Val Val Ala Ser Thr Ser Ser
65                  70                  75                  80

Ala Ala Ser Glu Ile Leu Lys Thr His Asp Arg Ile Leu Ser Ala Arg
                85                  90                  95

Tyr Val Phe Gln Ser Phe Arg Val Lys Gly His Val Glu Asn Ser Ile
            100                 105                 110

Val Trp Ser Asp Cys Thr Glu Thr Trp Lys Asn Leu Arg Lys Val Cys
        115                 120                 125

Arg Thr Glu Leu Phe Thr Gln Lys Met Ile Glu Ser Gln Ala His Val
    130                 135                 140

Arg Glu Lys Lys Cys Glu Glu Met Val Glu Tyr Leu Met Lys Lys Gln
145                 150                 155                 160

Gly Glu Glu Val Lys Ile Val Glu Val Ile Phe Gly Thr Leu Val Asn
                165                 170                 175

Ile Phe Gly Asn Leu Ile Phe Ser Gln Asn Ile Phe Glu Leu Gly Xaa
            180                 185                 190

Pro Asn Ser Gly Ser Ser Glu Phe Lys Glu Tyr Leu Trp Arg Met Leu
        195                 200                 205

Glu Leu Gly Asn Ser Thr Asn Pro Ala Asp Tyr Phe Pro Met Leu Gly
    210                 215                 220

Lys Phe Asp Leu Phe Gly Gln Arg Lys Glu Val Ala Glu Cys Leu Lys
225                 230                 235                 240

Gly Ile Tyr Ala Ile Trp Gly Ala Met Leu Gln Glu Arg Lys Leu Ala
                245                 250                 255

Lys Lys Val Asp Gly Tyr Gln Ser Lys Asn Asp Phe Val Asp Val Cys
            260                 265                 270

Leu Asp Ser Gly Leu Asn Asp Tyr Gln Ile Asn Ala Leu Leu Met Glu
        275                 280                 285

Leu Phe Gly Ala Gly Thr Glu Thr Ser Ala Ser Thr Ile Glu Trp Ala
    290                 295                 300

Met Thr Glu Leu Thr Lys Asn Pro Lys Ile Thr Ala Lys Leu Arg Ser
305                 310                 315                 320

Glu Leu Gln Thr Val Val Gly Glu Arg Ser Val Lys Glu Ser Asp Phe
                325                 330                 335

Pro Asn Leu Pro Tyr Leu Glu Ala Thr Val Lys Glu Thr Leu Arg Leu
            340                 345                 350

His Pro Pro Thr Pro Leu Leu Leu Pro Arg Arg Ala Leu Glu Thr Cys
        355                 360                 365

Thr Ile Leu Asn Tyr Thr Ile Pro Lys Asp Cys Gln Ile Met Val Asn
    370                 375                 380

Ala Trp Gly Ile Gly Arg Asp Pro Lys Thr Trp Ile Asp Pro Leu Thr
385                 390                 395                 400

Phe Ser Pro Glu Arg Phe Leu Asn Ser Ser Val Asp Phe Arg Gly Asn
                405                 410                 415

Asp Phe Ser Leu Ile Pro Phe Gly Ala Gly Arg Arg Ile Cys Pro Gly
            420                 425                 430

Leu Pro Ile Ala Asn Gln Phe Ile Ala Leu Leu Val Ala Thr Phe Val
        435                 440                 445

Gln Asn Leu Asp Trp Cys Leu Pro Asn Gly Met Ser Val Asp His Leu
    450                 455                 460

Ile Val Glu Glu Lys Phe Gly Leu Thr Leu Gln Lys Glu Pro Pro Leu
465                 470                 475                 480

Phe Ile Val Pro Lys Ser Arg Val
                485

<210>  9
<211>  1613
<212>  DNA
<213>  Papaver somniferum

<220>
<221>  misc_feature
<222>  (612)..(612)
<223>  N = unknown

<400>  9
aagtccgagt gagaagcaga gttagagaaa aaaaaaatgg aggttgtcac agtagcactt      60

attgcagtaa taatttcttc aatactctac ctcctctttg gtagtagtgg tcacaaaaat     120

ctcccaccag gaccaaaacc atggccaata gtaggaaatc ttctccaact tggtgagaaa     180

ccacacgctc aattcgccga actagctcaa acctatggtg acattttcac tcttaaaatg 240
ggtactgaaa ctgtagttgt tgcatcaaca tcttcggcag cttccgaaat actaaaaacc 300
catgatcgaa ttctatccgc tcgttacgtt tttcaaagtt ttcgagtaaa agggcatgta 360
gaaaattcaa tagtttggtc agattgtact gaaacttgga agaatttaag aaaagtttgt 420
aggacggaac ttttcacaca gaagatgata gaaagtcaag ctcatgttag agagaaaaaa 480
tgtgaagaaa tggttgaata cttgatgaaa aaacaagggg aagaagtgaa aattgtggaa 540
gtaatatttg gaacattagt gaatatattc ggaaatttga tattttcaca gaatatattt 600
gaattgggtg anccaaatag tggaagttca gagttcaagg aatatctatg gaggatgttg 660
gaattaggga attcaacaaa tccagctgat tattttccaa tgttgggtaa atttgatttg 720
tttggacaga ggaaagaagt tgcagagtgt ttaaaaggga tttatgctat ttggggagct 780
atgcttcaag aaaggaaatt agctaaaaaa gttgatggat accaaagcaa gaatgatttt 840
gttgatgttt gtcttgattc tggacttaat gattatcaga tcaatgcctt gcttatggaa 900
ttatttgggg caggcacaga aacaagcgca tcgacaattg agtgggccat gactgaacta 960
acaaagaatc caaagataac agctaagctt agatcagaac ttcaaacagt ggtaggcgag 1020
agatcggtaa aagaatccga cttccccaat cttccatacc ttgaagctac tgttaaagaa 1080
accctaagac ttcacccacc aactccattg ctactcccac gtcgagcact tgaaacctgt 1140
acaatcctca actacaccat cccaaaagat tgtcaaatta tggtcaacgc ttggggaatc 1200
ggacgtgatc ccaagacttg gatcgatccg ttgactttct caccagagag attcttgaat 1260
tctagtgttg actttagggg gaatgatttc agtttgatac catttggtgc aggaagaagg 1320
atatgccccg gtctgccaat agcaaatcag tttattgcat tgctagtggc aacatttgtg 1380
caaaatttgg attggtgtct accaaatggg atgagtgttg accatttgat agtggaggag 1440
aagtttgggt tgactcttca aaaagaacca cctctattca ttgttcctaa atcaagggtt 1500
tgatcttctc atcatggttc atgtgaaaac tagattatct attgtgaagg ctatgtatgt 1560
tttctttcct tcctataaaa aaaaaaaaaa aaaaaaaaaa aaaaaaaaaa aaa 1613